(19) **Europäisches Patentamt**
**European Patent Office**
**Office européen des brevets**

(11) **EP 3 175 776 B1**

(12) **EUROPEAN PATENT SPECIFICATION**

(45) Date of publication and mention
of the grant of the patent:
**30.09.2020 Bulletin 2020/40**

(51) Int Cl.:
*A61B 3/028* (2006.01)     *A61B 3/103* (2006.01)
*A61B 3/032* (2006.01)

(21) Application number: **16201621.6**

(22) Date of filing: **01.12.2016**

(54) **OPTOMETRY APPARATUS AND OPTOMETRY PROGRAM**

OPTOMETRIEVORRICHTUNG UND OPTOMETRIEPROGRAMM

APPAREIL D'OPTOMÉTRIE ET PROGRAMME D'OPTOMÉTRIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.12.2015 JP 2015235229
01.12.2015 JP 2015235230
01.12.2015 JP 2015235231**

(43) Date of publication of application:
**07.06.2017 Bulletin 2017/23**

(73) Proprietor: **Nidek Co., Ltd.
Aichi 433-0038 (JP)**

(72) Inventors:
• **HORINO, Taeko
Gamagori-shi, Aichi 443-0038 (JP)**

• **SEKO, Jouji
Gamagori-shi, Aichi 443-0038 (JP)**
• **OGINO, Takeo
Gamagori-shi, Aichi 443-0038 (JP)**

(74) Representative: **Zimmermann, Tankred Klaus et al
Schoppe, Zimmermann, Stöckeler
Zinkler, Schenk & Partner mbB
Patentanwälte
Radlkoferstrasse 2
81373 München (DE)**

(56) References cited:
**JP-A- H05 253 186    JP-A- H08 238 220
JP-A- H11 285 470    US-A- 5 844 661
US-A1- 2005 105 050**

**Description**

BACKGROUND

1. Technical Field

The present disclosure relates to an optometry apparatus and optometry program for examining the examinee's eye.

2. Description of the Related Art

The following optometry apparatus is known. In this apparatus, in each of a pair of right and left lens units, a plurality of rotating discs with various optical elements disposed thereon is provided. By rotating the rotating discs, an optical element having desired optical characteristics is switched and disposed in an optometry window, and then the refractive power of the examinee's eye is subjectively examined.

During a corrected refractive power examination using this type of optometry apparatus, the right and left eyes are separately examined. The eye being examined (hereafter referred to as the measured eye) is presented with an examination target via the optical element disposed in the optometry window. In the examination window corresponding to the eye which is not being examined (hereafter referred to as the non-measured eye), a blocking plate provided on one of the rotating discs is disposed. Accordingly, the non-measured eye is not presented with the examination target.

Another method is known by which the blocking plate is not disposed on the non-measured eye side. In this method, the non-measured eye is fogged by, for example, adding a plus spherical power. Then, an "open-field" examination is implemented.

However, because the view in a corrected state varies from one examinee to another, fogging the non-measured eye sometimes requires adjustments to the added power.

An example of the relevant technology is disclosed in JP-A-11-285470.

JP H11 285470 A discloses an ophthalmoscope with a rotating disk where an optical element is arranged to correct an examined eye. A driving means to drive the rotating disk and arrange a lens of an intended correction diopter are provided in a pair of right and left lens units.

JP H11 285486 A discloses an apparatus such that an optical member of an optometry unit is changed by a controller.

JP H08 238220 A discloses an apparatus such that refractive power measurement is performed in an open-field state.

SUMMARY

In view of the problem of the related art, the present disclosure addresses the technical problem of providing an optometry apparatus and optometry program with which, when an open-field monocular examination is performed, a fogging state suitable to the examinee can be easily set.

In order to solve the problem, aspects of the present disclosure are provided with the following configurations.

The present invention is defined in the independent claims.

An optometry apparatus according to a first aspect of the present invention includes: a pair of right and left lens units disposed in an optical path of a target light flux projected onto an examinee's eye, for changing an optical characteristic of the target light flux; a drive means for driving the lens units; a control means for controlling driving of the drive means; and an acquisition means for acquiring a targeted visual acuity value at the time of fogging. The control means determines an amount of fog for fogging the examinee's eye on the basis of the targeted visual acuity value acquired by the acquisition means.

An optometry apparatus according to a second aspect is the optometry apparatus according to the first aspect configured such that the acquisition means additionally acquires a visual acuity value of the examinee's eye, and the control means determines the amount of fog on the basis of the targeted visual acuity value and the visual acuity value of the examinee's eye acquired by the acquisition means.

An optometry apparatus according to a third aspect is the optometry apparatus according to the first or second aspect further including an operation means. The acquisition means acquires the targeted visual acuity value on the basis of an operation signal output from the operation means in accordance with an operation by the examiner.

An optometry apparatus according to a fourth aspect is the optometry apparatus according to any one of the first to third aspects configured such that the pair of right and left lens units each includes at least an auto cross cylinder lens, the pair of right and left lens units changes the optical characteristic of the target light flux travelling on a first optical path serving as an optical path of the target light flux projected onto the examinee's right eye, and a second optical path serving as an optical path of the target light flux projected onto the examinee's left eye with the auto cross cylinder lens, and the control means controls the driving of the drive means so that separate directions of the examination target observed via the auto cross cylinder lens disposed in each of the first optical path and the second optical path become the same direction between the first optical path and the second optical path.

An optometry apparatus according to a fifth aspect is the optometry apparatus according to the fourth aspect configured such that the control means controls the driving of the drive means so that the axes of the auto cross cylinder lenses have the same axial angle between the first optical path and the second optical path.

An optometry apparatus according to a sixth aspect is the optometry apparatus according to the fourth or fifth aspect configured such that the drive means further includes a rotation drive means for modifying the respective rotation angles of the auto cross cylinder lenses respectively disposed in the first optical path and the second optical path, and the control means, by controlling the driving of the rotation drive means, rotates the auto cross cylinder lenses in conjunction with each other so that the axial angles of the auto cross cylinder lenses respectively disposed in the first optical path and the second optical path are maintained to be the same between the first optical path and the second optical path.

An optometry apparatus according to a seventh aspect is the optometry apparatus according to the sixth aspect configured such that the control means, when rotating the auto cross cylinder lenses using the rotation drive means, aligns the rotation directions and rotation amounts of the auto cross cylinder lenses respectively disposed in the first optical path and the second optical path.

An optometry apparatus according to an eighth aspect is the optometry apparatus according to the sixth or seventh aspect configured such that the control means, when rotating the auto cross cylinder lenses using the rotation drive means, aligns the rotational speeds of the auto cross cylinder lenses respectively disposed in the first optical path and the second optical path.

An optometry apparatus according to a ninth aspect is the optometry apparatus according to any one of the first to eighth aspects further including a display means for displaying optometry information of the examinee's eye. The control means causes the display means to display a binocular simulation image indicating a view of the examination target as viewed by both eyes.

An optometry apparatus according to a tenth aspect is the optometry apparatus according to the ninth aspect configured such that the control means causes the display means to additionally display a monocular simulation image being at least one of a right-eye simulation image indicating a view of the examination target as viewed by the right eye, and a left-eye simulation image indicating a view of the examination target as viewed by the left eye.

An optometry apparatus according to a eleventh aspect is the optometry apparatus according to the tenth aspect configured such that the control means sets sharpness of at least one of the binocular simulation image and the monocular simulation image on the basis of the visual acuity value of the examinee's eye.

An optometry apparatus according to a twelfth aspect is the optometry apparatus according to the eleventh aspect configured such that the visual acuity value of the examinee's eye is an estimated visual acuity value of the examinee's eye being fogged.

An optometry apparatus according to a thirteenth aspect is the optometry apparatus according to any one of the ninth to twelfth aspects configured such that the control means sets sharpness of the binocular simulation image based on the higher of the right and left monocular visual acuity values.

An optometry apparatus according to a fourteenth aspect is the optometry apparatus according to any one of the ninth to thirteenth aspects further including a corrective means for correcting the examinee's eye. The control means changes the sharpness of the simulation image in accordance with a power change in a corrective optical system of the corrective means.

An optometry program according to a fifteenth aspect of the present invention, by being executed by a processor of an optometry apparatus, causes the optometry apparatus to implement: an acquisition step of acquiring a targeted visual acuity value at the time of fogging and a measured visual acuity value of the examinee's eye; and a determination step of determining, on the basis of the targeted visual acuity value and the measured visual acuity value, an amount of fog to be added when fogging the examinee's eye.

BRIEF DESCRIPTION OF THE DRAWINGS

FIG. 1 is an overall schematic diagram of an optometry system according to an example of the present disclosure.
FIG. 2 is an exterior schematic diagram of the optometry apparatus of the present example as viewed from the examinee side.
FIG. 3 is a horizontal cross sectional diagram of the optometry apparatus of the present example.
FIG. 4 is a diagram for describing an auto cross cylinder lens.
FIG. 5 is a control-system block diagram of the present example.

FIG. 6 is a flowchart of an operation of the present example.

FIG. 7 is a diagram illustrating a fog-parameter setting screen.

FIG. 8 is a diagram for describing the amount of fog computation.

FIGS. 9A to 9C are diagrams for describing the view of a point group chart.

FIGS. 10A to 10C are diagrams for describing the view of a point group chart.

FIG. 11 is a diagram for describing rotation control of the auto cross cylinder lens.

FIG. 12 is a diagram illustrating an example of a display screen of a controller.

FIGS. 13A to 13C are diagrams for describing the view of the target at the time of fogging.

FIGS. 14A and 14B are diagrams for describing an eye chart.

## DESCRIPTION OF THE EMBODIMENTS

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

In the following, a first embodiment according to the present disclosure will be briefly described. According to the first embodiment, an optometry apparatus (for example, an optometry apparatus 100) examines the examinee's eye. The optometry apparatus may be provided with a lens unit (for example, a lens unit 110); an acquisition unit (acquisition means; such as a control unit 370); a drive unit (drive means; such as drive units 150R and 150L); and a control unit (control means; such as a control unit 370), for example. The lens unit may include a pair of right and left lens units, for example. The lens unit is disposed in the optical path of a target light flux projected onto the examinee's eye (examinee), for example. The lens unit changes the optical characteristics of the target light flux, for example. The lens unit may include an optometry window (for example, the optometry window 160), for example. The lens unit may switch and dispose a plurality of lenses in the optometry windows. The lens unit may select the lens to be disposed in the optometry windows from among the plurality of lenses. The plurality of lenses may include a spherical lens, a cylinder lens, and an auxiliary lens.

The acquisition unit acquires a targeted visual acuity value when the examinee's eye (for example, the eye which is not being measured (referred to as the non-measured eye)) is fogged (at the time of fogging). The fogging of the examinee's eye (for example, the non-measured eye) may involve, for example, disposing a lens that blurs the examinee's eye on the visual line of the examinee's eye (examination window). The targeted visual acuity value may be the visual acuity value of the non-measured eye being fogged, for example. As a result of fogging the non-measured eye, the visual acuity of the non-measured eye may be limited. In this case, an examination of the measured eye (for example, visual acuity examination) may be performed with the non-measured eye being fogged. The acquisition unit may acquire the targeted visual acuity value on the basis of an operation signal output from an operating unit (for example, a controller 300) in accordance with an operation by the examiner.

The drive unit drives the lens unit, for example. The control unit controls the driving of the drive unit, for example. For example, the control unit may control the driving of the drive unit with regard to the switching of the lens to be disposed in the optometry window, rotation of the lens disposed in the optometry window, or the like. For example, the control unit may determine the amount of fog for fogging the examinee's eye (for example, the non-measured eye) on the basis of the targeted visual acuity value acquired by the acquisition unit. In this way, for example, the examiner can easily perform an examination of the measured eye (for example, a visual acuity examination) in a state in which the non-measured eye is being fogged so that the visual acuity value of the non-measured eye has a desired visual acuity value. The amount of fog may be the power (diopter) of the lens disposed in the examination window for the examinee's eye (for example, the non-measured eye) at the time of fogging, for example.

The acquisition unit may further acquire the visual acuity value of the examinee (the examinee's eye), for example. The visual acuity value of the examinee may be a visual acuity value acquired by subjective examination, or a visual acuity value estimated in accordance with the corrected state of the examinee's eye. The acquisition unit may acquire, as the visual acuity value of the examinee's eye, a visual acuity value obtained by examining the eyes one by one, or a visual acuity value obtained by examining both eyes simultaneously. In this case, the control unit may determine the amount of fog on the basis of the targeted visual acuity value acquired by the acquisition unit and the visual acuity value of the examinee (the examinee's eye), such as the visual acuity value of the non-measured eye.

According to the invention, the control unit may determine the amount of fog on the basis of a power corresponding to the targeted visual acuity value and a correction value corresponding to the visual acuity value of the examinee. The correction value is a power obtained by conversion of the degree of blurring corresponding to the visual acuity value of the examinee. According to the invention, the correction value is a value obtained by converting a displacement between the image forming position of the examination target that enters the examinee's eye that has been corrected and the retinal position of the examinee's eye into a lens power.

When the visual acuity value of the examinee is acquired by the acquisition unit, the control unit may correct the amount of fog when fogging the examinee's eye (for example, the non-measured eye), in accordance with the visual acuity value of the examinee acquired by the acquisition unit. For example, the control unit may correct a predetermined amount of fog in accordance with the visual acuity value of the examinee.

The control unit may execute an optometry program stored in a storage unit (for example, a storage unit 340) so as to cause the optometry apparatus to implement the acquisition step and the determination step. The acquisition step is the step of acquiring the targeted visual acuity value at the time of fogging and the visual acuity value of the examinee, for example. The determination step is the step of determining, on the basis of the targeted visual acuity value and the measured visual acuity value, the amount of fog to be added for fogging the examinee's eye (for example, the non-measured eye), for example.

The optometry apparatus of the first embodiment may perform an open-field monocular examination by disposing a prism lens with respect to the non-measured eye (the examination window for the non-measured eye) so as to remove the visual line of the non-measured eye from the examination target. In this case, the acquisition unit may acquire a prism value of the examinee (for example, a prism value of the non-measured eye). In addition, the control unit may determine a prism value of the prism lens disposed with respect to the examinee's eye (for example, the non-measured eye) in accordance with the prism value of the examinee acquired by the acquisition unit. In this way, the optometry apparatus can reliably remove the visual line of the non-measured eye from the examination target.

In the following, a second embodiment of the present disclosure will be briefly described. According to the second embodiment, an optometry apparatus (for example, an optometry apparatus 100) is provided with, for example, a lens unit (for example, the lens unit 110); a drive unit (for example, drive units 130R, 130L, 150R, and 150L); and a control unit (for example, a control unit 370). The lens unit is disposed in each of a first optical path and a second optical path, for example. The first optical path is the optical path of a target light flux projected onto the examinee's right eye, for example. The second optical path is the optical path of a target light flux projected onto the examinee's left eye, for example. The lens unit may be provided with at least an auto cross cylinder lens, for example. In this case, the lens unit, using at least the auto cross cylinder lens, changes the optical characteristics of the target light flux in the first optical path and the second optical path respectively, for example. The auto cross cylinder lens separates the visual field of the examinee's eye using a prism component, for example. For example, the auto cross cylinder lens separates the visual field of the examinee's eye so that one examination target is visible doubly. The examiner, by having the examinee compare the view of the doubly separated examination targets, performs an examination (for example, a cross cylinder test) of the astigmatism direction and astigmatism power of the examinee's eye.

The drive unit may rotationally drive the lenses disposed in the first optical path and the second optical path respectively, for example. The control unit controls the driving of the drive unit, for example. For example, the control unit may control the driving of the drive unit so that the separate directions of the examination targets separated by the auto cross cylinder lens respectively disposed in the first optical path and the second optical path become the same direction between the first optical path and the second optical path. For example, the control unit may cause the auto cross cylinder lenses to be respectively disposed in the first optical path and the second optical path so that the axes of the auto cross cylinder lenses have the same axial angle between the first optical path and the second optical path. In this way, the separate directions of the target between the measured eye and the non-measured eye being fogged become the same. As a result, an open-field examination can be performed properly.

The drive unit may be provided with a rotational drive unit (rotation drive means; such as drive units 150R and 150L). The rotational drive unit modifies the rotation angle of the lenses (for example, auto cross cylinder lenses) disposed in the first optical path and the second optical path respectively. In this case, the control unit, by controlling the driving of the rotational drive unit, may cause the auto cross cylinder lenses to rotate in conjunction so that the axial angles of the auto cross cylinder lenses disposed in the first optical path and the second optical path are maintained to be the same between the first optical path and the second optical path. For example, the control unit, when rotating the auto cross cylinder lenses via the rotational drive unit, may align the directions and amounts of rotation of the auto cross cylinder lenses disposed in the first optical path and the second optical path.

The control unit, when rotating the auto cross cylinder lenses via the rotational drive unit, may align the rotational speed of the auto cross cylinder lenses disposed in the first optical path and the second optical path.

A processor of the control unit may execute an optometry program stored in a storage unit (for example, the storage unit 340) so as to cause the control step to be implemented by the optometry apparatus. The control step is, for example, the step of controlling the driving of the lens unit so that, by the lens unit, the separate directions of the examination targets observed via the auto cross cylinder lens become the same direction between the first optical path and the second optical path. The auto cross cylinder lens is disposed in each of the first optical path, which is the optical path of the target light flux projected onto the examinee's right eye, and the second optical path, which is the optical path of the target light flux projected onto the examinee's left eye.

The lens unit may be provided with an optometry window (for example, the optometry window 160), for example. For example, the examinee sees the examination target through the optometry window. In this case, a plurality of lenses including an auto cross cylinder lens may be switched and disposed in a right optometry window (for example, right optometry window 160R) on the first optical path, and a left optometry window (for example, left optometry window 160L) on the second optical path.

Even in the case of a lens, such as the auto cross cylinder lens, including a prism component by which the examination target is separated into at least two parts, the control unit may control the driving of the drive unit so that the separate directions of the examination targets observed via the lenses respectively disposed in the first optical path and the second optical path become the same direction between the first optical path and the second optical path. In this case, the optometry apparatus according to the second embodiment may be expressed as follows. That is, the optometry apparatus of the second embodiment is an optometry apparatus for examining the examinee's eye, and is provided with a pair of right and left lens units; a drive unit for driving the lens units; and a control unit for controlling the driving of the drive unit. The pair of right and left lens units are respectively disposed in a first optical path which is the optical path of a target light flux projected onto the examinee's right eye, and a second optical path which is the optical path of a target light flux projected onto the examinee's left eye. The pair of right and left lens units, using a lens including at least a prism component, changes the optical characteristics of the target light flux in the first optical path, and changes the optical characteristics of the target light flux in the second optical path. The control unit controls the driving of the drive unit so that the separate directions of examination targets, observed via lenses which are respectively disposed in the first optical path and the second optical path and which include a prism component, become the same direction between the first optical path and the second optical path.

In the following, a third embodiment according to the present disclosure will be briefly described. According to the third embodiment, an optometry apparatus is provided with a display unit for displaying optometry information of the examinee's eye (display means; for example, a display unit 320 of a display terminal such as the controller 300). The optometry information may include an examination status and examination results of the examinee's eye, for example. The control unit causes the display unit to display a binocular simulation image, for example. The binocular simulation image shows by itself the view of the examination target by both eyes. The binocular simulation image may be one (single) image, for example. The binocular simulation image may be a single simulation image based on the assumption that the examination target is being viewed by the measured eye that has been corrected by the lens unit and the like, and the non-measured eye that has been fogged by the lens unit and the like, for example. That is, the binocular simulation image may be a single simulation image obtained by the merging in the brain of the retinal images which are respectively obtained by the left and right eyes when the examination target is viewed by the measured eye corrected by the lens unit and the like, and by the non-measured eye fogged by the lens unit and the like. For example, when the non-measured eye is fogged, the difference in the view between the measured eye and the non-measured eye is large. Accordingly, it is not easy for the examiner to know the view by both eyes. Even in such a case, the display of the binocular simulation image allows the examiner to know the view by both eyes easily.

The control unit may further cause the display unit to display a monocular simulation image, for example. The monocular simulation image is at least one of the right-eye simulation image and the left-eye simulation image, for example. For example, the right-eye simulation image is an image indicating the view of the examination target as viewed by the right eye. For example, the left-eye simulation image is an image indicating the view of the examination target as viewed by the left eye. The display of a binocular simulation image and a monocular simulation image allows the examiner to know the relationship between the view by one eye and the view by both eyes easily.

The control unit may set the sharpness of at least one of the binocular simulation image and the monocular simulation image on the basis of the visual acuity value of the examinee's eye. The visual acuity value of the examinee's eye may be a value obtained through a visual acuity examination, or a value input by the examiner as desired. Alternatively, the visual acuity value of the examinee's eye may be, for example, an estimated value based on a corrected state of the examinee. For example, the visual acuity value of the examinee's eye may be an estimated visual acuity value of the examinee's eye being fogged, or a visual acuity value estimated on the basis of the corrective information of the examinee's eye being corrected by a corrective unit (for example, the lens unit 110). The sharpness herein may refer to at least any of the degree of blurring, sharpness of color, contrast, resolution, and brightness and the like of an image.

The control unit may set the sharpness of the binocular simulation image on the basis of the higher of the right and left monocular visual acuity values. Generally, in the case of the view by both eyes, priority is in many cases given to the view by one of the left and right eyes that provides a sharper view. Accordingly, the control unit may determine the sharpness of the binocular simulation image on the basis of the view by the eye with higher visual acuity.

The optometry apparatus may be additionally provided with a corrective unit (corrective means; such as the lens unit 110) for correcting the examinee's eye. In this case, the control unit may change the sharpness of at least one of the binocular simulation image and the monocular simulation image, in accordance with a change in power of the corrective optical system of the corrective unit. In this way, the examiner can easily know a change in the corrected state of the examinee and a change in the view as corrected.

The control unit may cause the display unit to display an eye chart. The eye chart, for example, is a diagram in which at least the examinee's eye and the image forming position of a target light flux entering the examinee's eye are drawn. The display of the eye chart allows the examiner to know the view by the examinee objectively. The eye chart may include a drawing of the corrective optical system disposed in front of the examinee's eye. The control unit may change the drawing of the eye chart in accordance with a change in the corrected state of the examinee's eye being corrected by the corrective unit, for example. For example, the control unit may change the image forming position of the target light flux, the shape of the corrective optical system, or the number of lenses.

The processor of the control unit may execute an optometry-information display program stored in the storage unit (for example, the storage unit 340) so as to cause a display step to be implemented by the optometry apparatus, for example. The display step, for example, is the step of causing the display unit to display the binocular simulation image indicating the view of the examination target by both eyes.

In the foregoing description of the embodiments, the lens unit of the optometry apparatus may be disposed on the examinee side (for example, in front of the examinee's eye), or on the target presentation apparatus side. For example, the lens unit may be disposed in the target presentation apparatus (for example, a target presentation apparatus 200) (so-called phantom type). In this case, the target presentation apparatus may be configured to project a target light flux with corrected optical characteristics onto the examinee's eye using the internally disposed lens unit. The target presentation apparatus herein refers to an apparatus for projecting the examination target onto the examinee, for example.

The lens unit for changing the optical characteristics of the target light flux is not limited to the configuration including a lens switch mechanism but may be a lens unit in which an Alvalez lens is disposed.

<Examples>

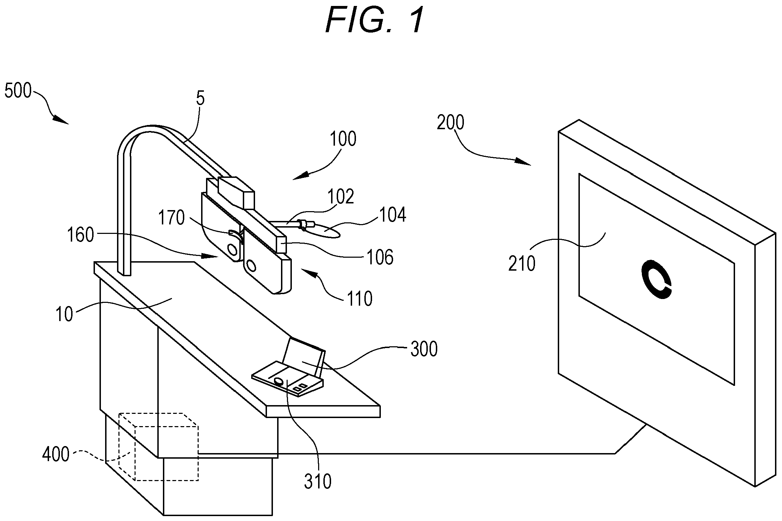

In the following, an optometry system 500 according to the present example will be described. As illustrated in FIG. 1, the optometry system 500 according to the present example is provided with the optometry apparatus 100; the target presentation apparatus 200; the controller 300; and the relay unit 400, for example. For example, the optometry apparatus 100 is hung from a holding arm 5. One end of the holding arm 5 is fixed to a base 10, for example.

<Optometry apparatus>

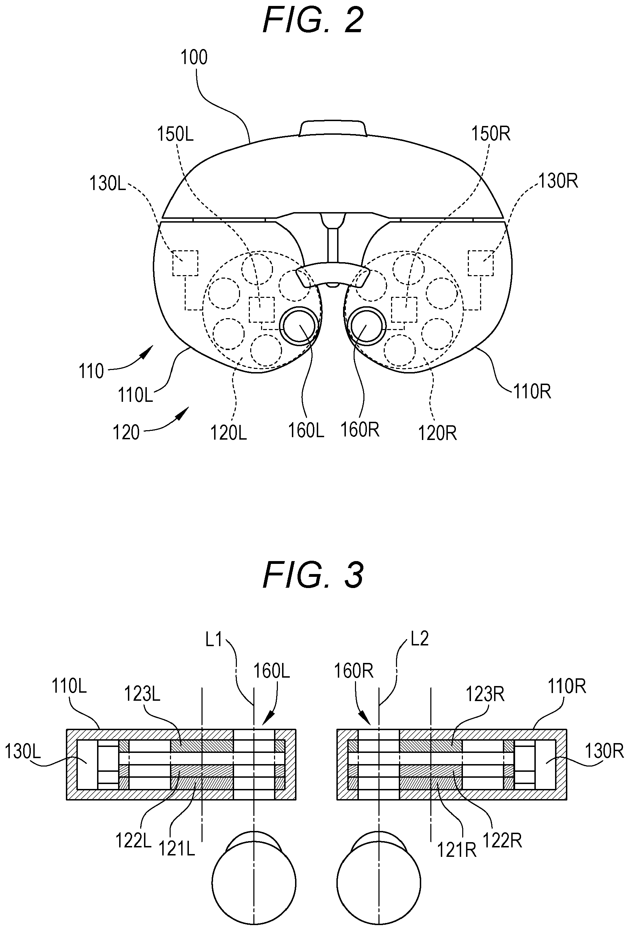

As illustrated in FIG. 2, the optometry apparatus 100 is provided with a pair of right and left lens units 110 (left lens unit 110L, right lens unit 110R). The lens unit 110 is provided with lens discs 120 (left lens discs 120L, right lens discs 120R). The lens discs 120 are rotatably held in the lens unit 110 (see FIG. 2). On the lens discs 120, a number of optical elements (for example, a spherical lens, a cylinder lens, and a dispersing prism) are disposed on the same circumference.

The rotation control of the lens discs 120 by the drive units (actuators) 130R, 130L enables the optical element desired by the examiner to be disposed in the optometry window 160 (left optometry window 160L, right optometry window 160R). In addition, the rotation control by the drive units 150R, 150R of the optical element (for example, a cylinder lens, a cross cylinder lens, or a rotary prism) disposed in the optometry window 160 enables the optical element to be disposed at a rotation angle desired by the examiner. The switching and the like of the optical element disposed in the optometry window 160 is performed by, for example, the examiner operating the controller 300 (see FIG. 1) serving as the operation means. Examples of the drive units 130R, 130L and the drive units 150R, 150L include motors and solenoids. However, the drive units 130R, 130L and the drive units 150R, 150L are not limited thereto.

The lens unit 110 may be provided with a plurality of lens discs 120 (120L, 120R). For example, each of the lens discs 120 is provided with an opening (or a 0D lens) and a plurality of optical elements. Examples of the type of the lens discs 120 include, as illustrated in FIG. 3, spherical lens discs 121R, 121L; cylinder lens discs 122R, 122L; and auxiliary lens discs 123R, 123L.

The spherical lens discs 121R, 121L include, for example, a plurality of spherical lenses with different powers. The cylinder lens discs 122R, 122L include, for example, a plurality of cylinder lenses with different powers. The auxiliary lens discs 123R, 123L have at least any of a red filter/green filter; a prism; a cross cylinder lens; a polarization plate; a Maddox lens; and an auto cross cylinder lens disposed thereon. The cylinder lens, the rotary prism, the auto cross cylinder lens and the like are disposed so as to be rotatable about an optical axis L1 or an optical axis L2 by the drive units 150R, 150L. The drive units 130R, 130L and the drive units 150R, 150L may be provided for each of the lens discs 120.

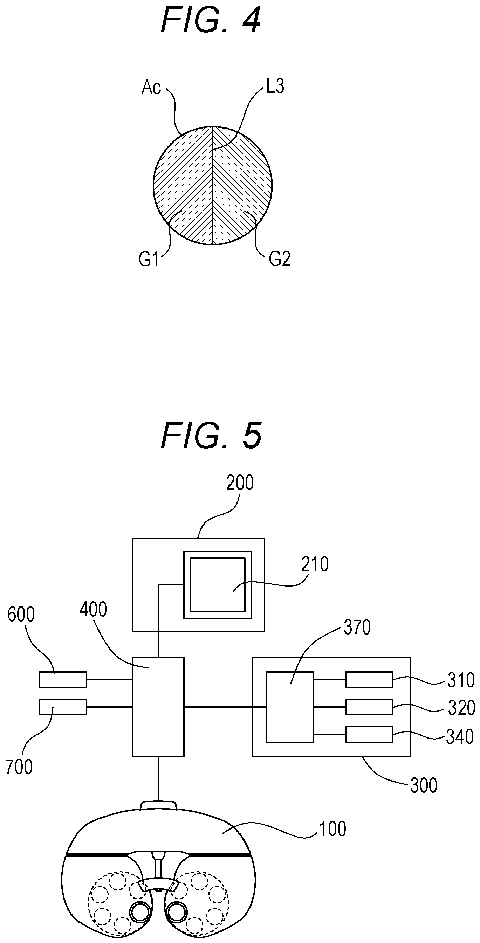

The auto cross cylinder lens is a lens for performing a cross cylinder (auto cross) test. The cross cylinder test is a test for measuring the direction and power of astigmatism of the examinee's eye. As illustrated in FIG. 4, for example, an auto cross cylinder lens Ac is provided with two prism regions G1, G2 by which the visual field is divided. The two prism regions G1, G2 are divided at the boundary of an axis L3, for example. In this case, for example, the visual field is divided along the vertical direction of the axis L3. In addition, each prism region is given an astigmatism power. The astigmatic axes of the prism regions are orthogonal to each other.

The optometry apparatus 100 may be provided with, e.g., a mechanism for adjusting the interval of the right and left lens units 110 and/or a mechanism for adjusting the convergence angle (inset angle) of the right and left lens units 110. As illustrated in FIG. 1, the optometry apparatus 100 may be provided with a forehead rest 170 which abuts on the examinee's forehead. The forehead rest 170 has the function of fixing the position of the examinee's eye at a predetermined examination position as well as holding the examinee's head.

The optometry apparatus 100 is not limited to the above configurations. The optometry apparatus 100 need only be configured to be able to switch electrically the optical elements disposed in front of the examinee's eye. For example, the optometry apparatus 100 may be configured to switch the power of the lens disposed in front of the examinee's eye, by the driving of an electric active lens.

<Target presentation apparatus>

The target presentation apparatus 200 is provided with a target presentation unit 210 for presenting the examination target to the examinee (the examinee's eye). The target presentation apparatus 200 is connected to the controller 300 via the relay unit 400. The switching and the like of the target displayed on the target presentation unit 210 are controlled by an operation of the controller 300 by the examiner, for example.

The target presentation apparatus 200 displays the examination target on the target presentation unit 210 in accordance with an operation signal input from the controller 300. The target presentation apparatus 200 is disposed at substantially the same height as the optometry apparatus 100. In addition, the target presentation apparatus 200 is installed away from the optometry apparatus 100 by a distance suitable for the examination. In the present example, the distance between the optometry apparatus 100 and the target presentation apparatus 200 is a distance suitable for distance examination (for example, 5 m). The target presentation apparatus 200 is not limited to the illustrated display, and may be a chart projector which projects the target on a screen, or a space-saving target projection apparatus which projects the target via a concave mirror.

As illustrated in FIG. 1, the optometry apparatus may be provided with a near target presentation unit 104 for performing a near examination. For example, the near target presentation unit 104 is slidably held on a rod 102 attached to the optometry apparatus. The near target presentation unit 104 is provided with a chart plate with a plurality of examination targets drawn thereon, or a liquid crystal display, for example.

<Controller>

The controller 300 illustrated in FIG. 1 operates (controls) at least one of the optometry apparatus 100 and the target presentation apparatus 200. The controller 300 includes an operation panel 310 with a plurality of operation buttons disposed thereon, and a display unit (display panel) 320 with a touch panel function. The controller 300 detects an operation by the examiner with respect to the operation panel 310 and the display unit (display panel) 320. The controller 300, on the basis of the operation by the examiner, outputs a drive signal to the optometry apparatus 100 and/or the target presentation apparatus 200. The controller 300 is used by the examiner to provide an instruction for switching the optical element disposed in front of the examinee's eye.

As illustrated in FIG. 5, the control unit 370 functions as a main execution entity of the controller 300. The control unit 370 executes various processes in accordance with a program for controlling the optometry apparatus 100 and the target presentation apparatus 200. Such program is stored in a storage unit 340 with which the controller 300 is provided, for example.

The control unit 370 for controlling the optometry apparatus 100 and the target presentation apparatus 200 and the like is not limited to the controller 300. The control unit 370 may be provided in the optometry apparatus 100, the target presentation apparatus 200, or the relay unit 400, for example.

<Relay unit>

The relay unit 400 illustrated in FIG. 1 is a unit for controlling the power supply to the optometry apparatus 100 and communication from the controller 300 to the optometry apparatus 100 and the target presentation apparatus 200. The relay unit 400 is connected to the controller 300, the optometry apparatus 100, and the target presentation apparatus 200. The mode of connection may be wireless or wired connection. The relay unit 400 is provided with, for example, a control unit including a CPU and the like. The relay unit 400 receives a control command from the controller 300 (control unit 370), and controls the optometry apparatus 100 and the target presentation apparatus 200. It should be noted that the relay unit 400 is not a necessarily required element. The optometry apparatus 100 and the target presentation apparatus 200 may be configured to receive the control command from the controller 300.

<Control operation>

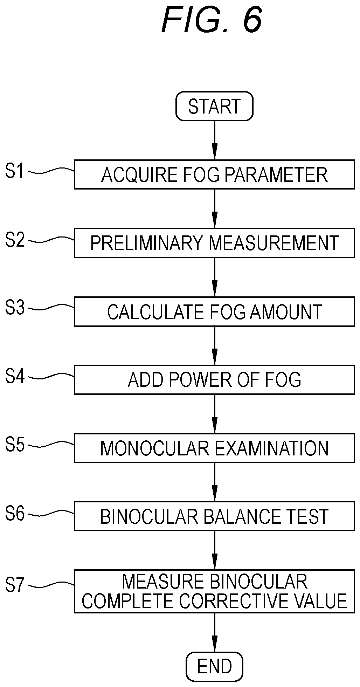

The operation of the apparatus with the above configuration will be described (see the examination procedure flowchart of FIG. 6). In the following, by way of example, a case will be described in which the non-measured eye is fogged at the monocular measurement and the examination is performed in an open-field state.

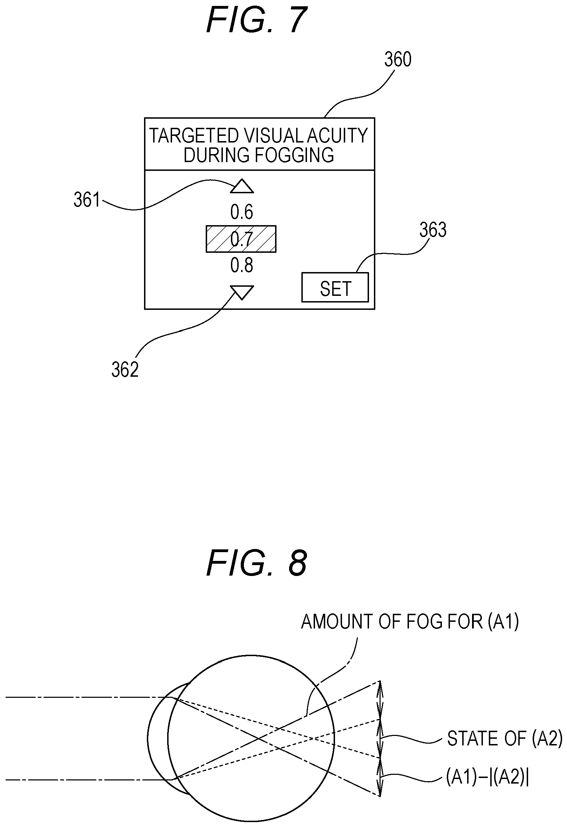

An examination performed in an open-field state, compared with when one eye is shielded with a blocking plate, is less susceptible to unwanted accommodation by the examinee's eye, so that an appropriate examination can be easily performed. When fogging is implemented, the examiner sets, prior to the examination, a fog parameter for determining the amount of fog. An example of the fog parameter is the targeted visual acuity value at the time of fogging. For example, the control unit 370 causes the display unit 320 to display a visual acuity-value setting screen 360 as illustrated in FIG. 7, and receives the input of a targeted visual acuity value from the examiner.

On the visual acuity-value setting screen 360, select buttons 361, 362 and a set button 363 are displayed, for example. In this case, the examiner, by operating the select button 361 or the select button 362, may select the targeted visual acuity value at the time of fogging. In addition, the examiner may input (set) the selected targeted visual acuity value by pressing the set button 363. The control unit 370 receives the input of the targeted visual acuity value, and causes the input targeted visual acuity value to be stored in the storage unit 340 and the like (step S1). The setting of the targeted visual acuity value may not necessarily be performed for each examination. For example, once the setting is made on the setting screen, the same targeted visual acuity value is used for the subsequent examinations.

After the targeted visual acuity value is set, the examiner starts examination on the examinee's eye. In the present example, prior to a subjective refractive power measurement, a preliminary examination is performed (step S2). The preliminary examination is implemented by, for example, an eye refractive power measurement apparatus 600 (see FIG. 5). The preliminary examination may include, for example, an objective measurement; a previous-spectacles measurement; a monocular cover test; an alternate cover test; a naked eye /previous-spectacles visual acuity confirmation; a balance test by polarizing RG (red/green); and a visual acuity measurement based on an objective value. When the examinee is wearing spectacles, the power of the spectacles lens (spectacles value data) may be measured using a lens meter 700. The results of the measurement may provide information for prescription. The respective items of measurement data may be input to the controller 300. For example, the measurement data are stored in the storage unit 340 and the like. The controller 300 may receive the results of measurement by the eye refractive power measurement apparatus 600, the lens meter 700 and the like via wireless or wired communication means. Alternatively, the examiner may input the measurement results to the controller 300.

In the optometry window 160 of the optometry apparatus 100, an optical system of an initial setting is disposed, for example. In this case, as the examiner calls objective value data that have been input in advance or the previous spectacles value data, a corrective optical system based on the data is disposed in the right and left optometry windows 160. As a result, a subjective examination can be performed with high efficiency. In the following description, a case in which objective value data are utilized will be described by way of example.

When the lenses based on the objective value data are disposed in the right and left optometry windows 160, the examiner confirms whether the view by the left and right eyes is appropriate by, for example, a polarizing R/G examination. As the examination target, a polarizing R/G examination target is used. In the polarizing R/G examination target displayed on the target presentation unit 210, for example, the polarizing directions are orthogonal to each other between the right-eye target and the left-eye target. For example, the polarizing direction for the right-eye target is set at 135°. The polarizing direction for the left-eye target is set at 45°. In the right and left optometry windows, the polarizing lenses provided on the auxiliary lens discs 123R, 123L are disposed such that the polarizing axes of the polarizing lenses respectively correspond to the right and left targets. The examiner, for example, confirms whether the respectively corresponding targets are being seen by the right and left eyes, and/or whether the balance in the view by the left and right eyes is appropriate. In this way, the examiner confirms whether an examination which uses fogging instead of a shield is appropriate to the examinee. For example, if, during the polarizing R/G examination, the examinee is unable to recognize both the right-eye target and the left-eye target visually, the examination using fogging instead of a shield is not appropriate to the examinee. In addition, the examiner confirms whether the corrective optical system values that have initially been set on the basis of objective value data are appropriate. Thereafter, the examiner confirms binocular visual acuity values by a visual acuity examination, for example. The control unit 370 causes the visual acuity values obtained by the visual acuity examination to be stored in the storage unit 340 and the like, for example.

Once the binocular visual acuity values have been confirmed, the control unit 370 calculates the fogging amount (the amount of fog) to be applied to the examinee's eye (for example, the non-measured eye) (step S3). For example, the control unit 370, in step S2, calculates the amount of fog on the basis of the visual acuity value of the examinee (the examinee's eye, such as the non-measured eye) stored in the storage unit 340 and the like, and a targeted visual acuity value input by the examiner prior to examination.

The control unit 370 may determine the amount of fog on the basis of tables such as Table 1 and Table 2. Table 1 shows the relationship between the targeted visual acuity value at the time of fogging and spherical power A1.

The spherical power A1 is added so as to fog the non-measured eye corrected with an auto refractometer value (objective measurement value) so that the visual acuity value of the non-measured eye becomes the targeted visual acuity value. Table 2 shows the correspondence relationship between the visual acuity value of the non-measured eye obtained by a visual acuity measurement with respect to the non-measured eye corrected with the auto refractometer value (visual acuity value of the non-measured eye corrected with the auto refractometer value), and the spherical power A2 corresponding to the degree of blurring in the view of the target to the non-measured eye having the visual acuity value (the degree of blurring after correction with the auto refractometer value). Table 2 may be considered to show the relationship between the visual acuity value of the non-measured eye corrected with the auto refractometer value, and the degree of blurring, corresponding to each visual acuity value, of the target in the non-measured eye corrected with the auto refractometer value.

Table 1

| Targeted visual acuity value | Spherical power A1(D) |
|---|---|
| 0.01 | 10.00 |
| 0.02 | 9.00 |
| 0.04 | 7.00 |
| 0.06 | 5.00 |
| 0.08 | 4.00 |
| 0.1 | 3.00 |
| 0.2 | 2.00 |
| 0.3 | 1.75 |
| 0.4 | 1.50 |
| 0.5 | 1.25 |
| 0.6 | 1.00 |
| 0.7 | 0.75 |
| 0.8 | 0.50 |
| 0.9 | 0.25 |
| 1.0 or above | 0 |

Table 2

| Visual acuity value of non-measured eye corrected with auto refractometer value | Spherical power A2 (D) corresponding to degree of blurring |
|---|---|
| 0.01 | ±10.00 |
| 0.02 | ±9.00 |
| 0.03 | ±8.00 |
| 0.04 | ±7.00 |
| 0.06 | ±5.00 |
| 0.08 | ±4.00 |
| 0.1 | ±3.00 |
| 0.2 | ±2.00 |
| 0.3 | ±1.75 |
| 0.4 | ±1.50 |
| 0.5 | ±1.25 |

(continued)

| Visual acuity value of non-measured eye corrected with auto refractometer value | Spherical power A2 (D) corresponding to degree of blurring |
| --- | --- |
| 0.6 | ±1.00 |
| 0.7 | ±0.75 |
| 0.8 | ±0.50 |
| 0.9 | ±0.25 |
| 1.0 or above | 0 |

For example, the control unit 370 may determine the amount of fog F by substituting a numerical value corresponding to the visual acuity value of the examinee (non-measured eye) and a numerical value corresponding to the fog parameter into the following expression (1). Expression (1) is an expression for determining the amount of fog F by subtracting, from the spherical power A1 corresponding to the targeted visual acuity value, the magnitude of the spherical power A2 corresponding to the degree of blurring after correction with the auto refractometer value.

$$F = (A1) - |(A2)| \qquad (1)$$

For example, when a targeted visual acuity value of 0.7 is input by the examiner, the control unit 370 applies fogging to the non-measured eye to such an extent that the target for visual acuity of 0.7 is visible. The control unit 370, using Table 1 for example, determines the spherical power A1 with respect to the visual acuity value of 0.7. In addition, the control unit 370, using Table 2, determines the spherical power A2 corresponding to the visual acuity value of the non-measured eye corrected with the auto refractometer value. The control unit 370 substitutes the spherical powers A1 and A2 into expression (1). In this case, if the visual acuity value (visual acuity measurement result) of the non-measured eye corrected with the auto refractometer value is 0.9, A1 = 0.75D and A2 = ±0.25. Accordingly, from expression (1), F = 0.50.

In this way, the control unit 370, when the visual acuity of the examinee (non-measured eye) is small (for example, less than 1.0), the target already appears blurred to the examinee (non-measured eye). Accordingly, the control unit 370 adds, to the non-measured eye, the amount of fog F obtained by subtracting the spherical power A2 from the spherical power A1 corresponding to the targeted visual acuity value at the time of fogging (see FIG. 8). That is, the control unit 370 determines the final amount of fog F by correcting a reference power (spherical power A1) with the correction power (spherical power A2). In the present example, the reference may be provided by the spherical power A1 for lowering the estimated visual acuity value (for example, 1.0 or more) for the non-measured eye corrected with the objective measurement value down to the targeted visual acuity value. Then, the spherical power A2 for correction may be determined on the basis of the difference between the actual visual acuity value of the examinee (non-measured eye) and the estimated visual acuity value of the non-measured eye corrected with the objective measurement value.

Obviously, the method for calculating the amount of fog F is not limited to the above example. For example, the control unit 370 may determine the spherical power A1 for obtaining the targeted visual acuity value as the amount of fog F, regardless of the visual acuity value of the non-measured eye corrected with the auto refractometer value. The control unit 370 may also fog the non-measured eye by adding not only a spherical power but also a cylindrical power.

Table 1 and Table 2 described above are examples, and the numerical values in the tables may be modified as desired. In the above example, the control unit 370 determines the amount of fog using Table 1 and Table 2. However, this is not a limitation, and the control unit 370 may determine the amount of fog by computation using a conversion formula and the like, without using tables.

The control unit 370 may add a certain amount of fog to the non-measured eye. For example, the control unit 370 may set a fixed value (for example, 0.3) as the targeted visual acuity value at the time of fogging. In this case, the control unit 370 may add a certain amount of fog to the non-measured eye so that the visual acuity value of the non-measured eye at the time of fogging becomes a fixed value that has been set.

If the auto refractometer value (or the visual acuity value of the non-measured eye corrected with the auto refractometer value) is not input, the control unit 370 may calculate the amount of fog by assuming a visual acuity value of the non-measured eye corrected with an auto refractometer value. For example, the control unit 370 may calculate the amount of fog by assuming that the visual acuity value of the non-measured eye corrected with an auto refractometer value is 1.0. When the visual acuity value of the non-measured eye corrected with the auto refractometer value is assumed to be 1.0, the spherical power A2 is 0D from Table 2. Accordingly, the amount of fog F is the same value as the spherical power A1 corresponding to the targeted visual acuity value.

When the amount of fog is determined, the control unit 370, prior to executing an examination using the fogging function, sends a command signal to the optometry apparatus 100 so as to add the amount of fog (for example, a plus spherical power) in addition to the spherical power of the lens already disposed in the optometry window 160. The control unit 370 adds the determined amount of fog by drivingly controlling the spherical lens disc 121R or the spherical lens disc 121L, for example (step S4). The adding of the amount of fog (for example, a plus spherical power) may include disposing a lens having a spherical power corresponding to the amount of fog in front of the non-measured eye.

In this way, the amount of fog is set by the control unit 370 automatically. Accordingly, the examiner is spared the need to operate the controller 300 to change the power of the lens discs 120. In addition, in the present example, the control unit 370 sets the amount of fog on the basis of the visual acuity value of the examinee. Accordingly, compared with when the amount of fog is set to a predetermined amount (for example, +1.00D), a fog state desired by the examiner can be appropriately reproduced. For example, even when the non-measured eye is corrected by an objective measurement value, a target with a visual acuity of 1.0 may be visible, or only a target with a visual acuity 0.9 may be visible, depending on the state of the examinee (non-measured eye). A difference in the view by the fogged non-measured eye is caused between adding a fog of +1.00D when the visual acuity of the non-measured eye is 1.0 and adding a fog of +1.00D when the visual acuity of the non-measured eye is 0.9. Specifically, adding a fog of +1.00D when the visual acuity of the non-measured eye is 0.9 causes a greater degree of blurring of the target for the non-measured eye than adding a fog of +1.00D when the visual acuity of the non-measured eye is 1.0. In the present example, the control unit 370 sets the amount of fog on the basis of the visual acuity value of the non-measured eye, whereby a fog state desired by the examiner can be achieved even in the presence of different visual acuity values and with respect to any examinee.

The examiner may set a plurality of fog parameters. The examiner, as fog parameters, may input a first targeted visual acuity value and a second targeted visual acuity value, for example. When a plurality of targeted visual acuity values are input as fog parameters, the control unit 370 may initially fog the non-measured eye so as to obtain the first targeted visual acuity value, and then change the target so that the second targeted visual acuity value can be obtained.

When the examinee's eye is fogged, a monocular measurement is implemented (step S5). For example, the examiner, by operating the controller 300, causes the examinee's both eyes to be fogged, and then only decreases the fogging on the measured eye. The examiner sets the power of fog on the measured eye to a minimum power such that the visual acuity of the measured eye is maximized. Thereafter, the examiner performs an R/G examination for preventing over-correction, a cross cylinder test (astigmatic axis examination and astigmatism power examination) and the like, and confirms the highest monocular visual acuity value through a visual acuity examination.

<Cross cylinder test>

A monocular examination of astigmatism power and astigmatic axis using an auto cross cylinder lens will be described. The examiner, by operating the controller 300, causes the target presentation apparatus 200 to display a point group chart. The control unit 370 then disposes the auto cross cylinder lens Ac in each of the optometry windows 160 for the measured eye and the non-measured eye. In this way, the auto cross cylinder lenses Ac are disposed in the right and left optometry windows 160, whereby an open-field cross cylinder test can be conducted using fogging even when an auto cross cylinder is used.

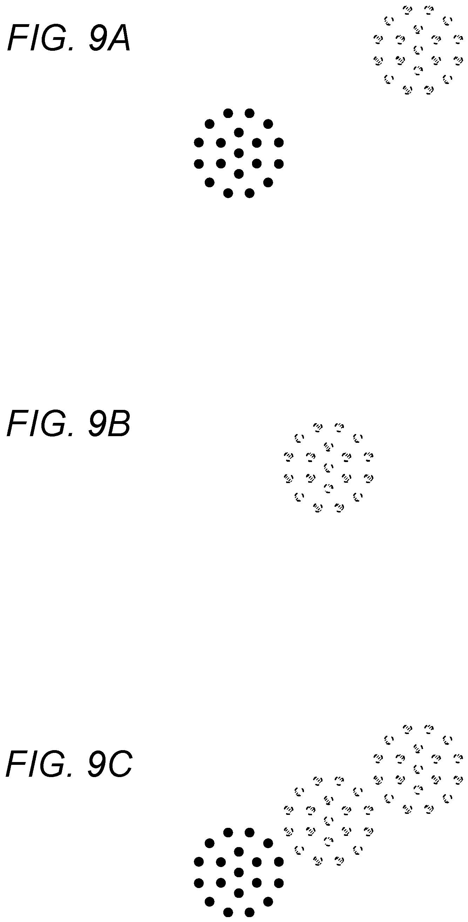

For example, when the auto cross cylinder lens is only disposed in the optometry window on the measured-eye side, as illustrated in FIG. 9A, the point group chart is visible doubly to the measured eye due to the prism component of the auto cross cylinder lens. As illustrated in FIG. 9B, to the non-measured eye, the point group chart is visible singly without being separated. In this case, the retinal images of the left and right eyes are not well merged, resulting in the point group chart being visible triply to the examinee, for example (see FIG. 9C). In this case, the examinee cannot tell which point group chart is being visually recognized by the measured eye, possibly resulting in a failure to perform an appropriate examination. By disposing the auto cross cylinder lenses Ac in the right and left optometry windows 160 as described above, the point group chart becomes separately visible to both eyes. In this way, an appropriate open-field cross cylinder test can be conducted.

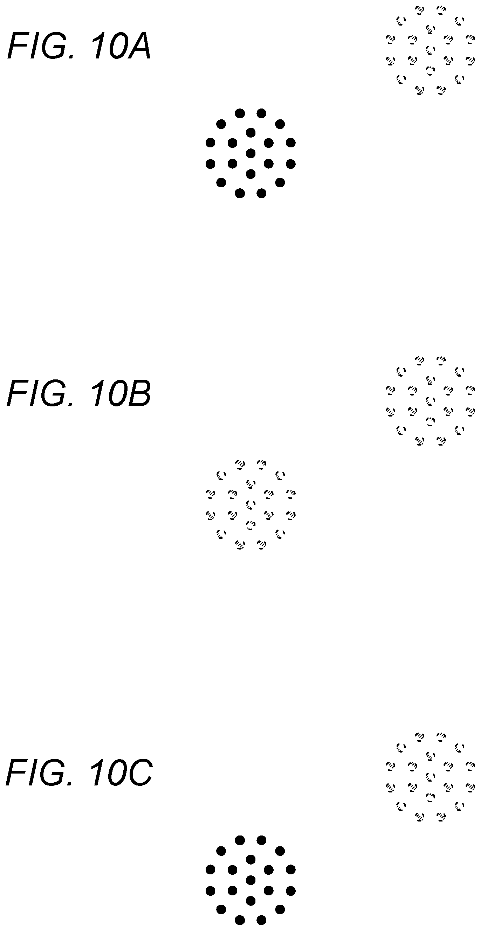

For example, as illustrated in FIG. 10A, to the measured eye, the point group chart is visible doubly due to the prism component of the auto cross cylinder lens. In addition, as illustrated in FIG. 10B for example, the point group chart is also visible doubly to the non-measured eye due to the prism component of the auto cross cylinder lens. Accordingly, as illustrated in FIG. 10C, the point group charts visible to the measured eye and the non-measured eye are appropriately merged. As a result, an appropriate cross cylinder test can be conducted.

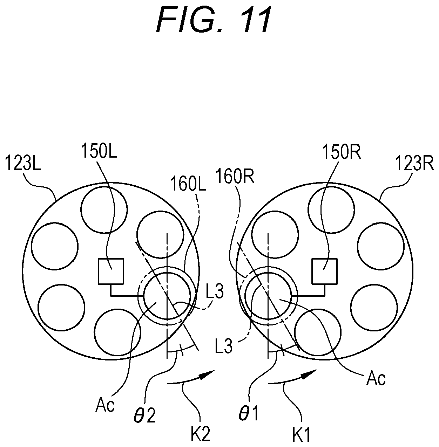

In this case, the control unit 370 disposes the right and left auto cross cylinder lenses Ac with the directions of the axes L3 of the auto cross cylinder lenses Ac aligned with each other. For example, the control unit 370 controls the angle of the axis L3 of the auto cross cylinder lens Ac on the non-measured eye side in accordance with the angle of the axis L3 of the auto cross cylinder lens Ac on the measured-eye side. For example, in the example illustrated in FIG. 11, the control unit 370 causes the angle θ1 of the axis L3 of the auto cross cylinder lens Ac disposed in the right optometry window 160R and the angle θ2 of the axis L3 of the auto cross cylinder lens Ac disposed in the left optometry window 160L to be aligned with each other. This is so that the separate directions of the point group chart divided in two by the prism of the auto cross cylinder lens Ac can be aligned. When the separate directions of the point group chart visible to the left eye and the right eye are aligned, the retinal images of the left and right eyes are well merged. As a result, an appropriate open-field examination can be conducted. In the illustrated example, the control unit 370 controls the angle θ1 and θ2 of the axes L3 with respect to the vertical direction. Instead, for example, the control unit 370 may control relative angles of the axes L3 of the right and left auto cross cylinder lenses Ac.

The examiner rotates the axis of the auto cross cylinder lens Ac until the difference in the view of the point group chart divided in two to the measured eye becomes small. In this case, the control unit 370 may rotate the auto cross cylinder lens Ac disposed on the measured-eye side and the auto cross cylinder lens Ac disposed on the non-measured eye side in synchronism with each other. For example, the control unit 370 may control the rotation of the auto cross cylinder lens Ac on the non-measured eye side in accordance with the rotation of the auto cross cylinder lens Ac on the measured-eye side. For example, in the example of FIG. 11, the control unit 370 aligns a rotation direction K1 of the axis L3 of the auto cross cylinder lens Ac disposed in the right optometry window 160R with a rotation direction K2 of the axis L3 of the auto cross cylinder lens Ac disposed in the left optometry window 160L. In addition, the control unit 370 may align the rotation amounts, rotational speeds, and the like of the right and left auto cross cylinder lenses Ac. In this way, the control unit 370, by controlling the drive units 150R, 150L, for example, causes the auto cross cylinder lenses Ac on the measured-eye side and the non-measured eye side to be rotated so that at least any of the rotation amounts, rotation directions, rotational speeds and the like of the auto cross cylinder lenses Ac on the measured-eye side and the non-measured eye side become the same. In this way, even when the auto cross cylinder lenses Ac are rotated, the separate directions of the point group chart as observed by each of the left and right eyes are aligned. Accordingly, the retinal images can be easily merged.

After the end of the astigmatic axis adjustment, the astigmatism power is adjusted. For example, the examiner switches the cylindrical power by driving the cylinder lens discs 122R, 122L until the difference in the view of the point group chart divided in two to the measured eye becomes small.

The control unit 370, after the end of monocular examination with respect to one of the eyes, switches the measured eye to the other eye. In this case, the control unit 370 removes the fog (the amount of fog) on the side of the eye to be measured (measured eye). The optical system on the measured-eye side becomes an optical system corresponding to the spherical power by the previously performed R/G examination. On the other hand, in the optical system on the non-measured eye side, a calculated amount of fog is added, as in step S4.

After the end of monocular examination with respect to each of the left and right eyes, the control unit 370 transitions to the next binocular balance examination (step S6). In this case, for example, the control unit 370 disposes the polarization plates of the auxiliary lens discs 123R, 123L in the right and left optometry windows, and removes the fog that has been applied to the non-measured eye. After the end of the binocular balance examination, the control unit 370 transits to the measurement of a binocular complete corrective value (step S7). For example, the control unit 370 once fogs both eyes, and measures a minimum power that provides the highest visual acuity. After the binocular complete corrective value is obtained, the control unit 370 ends the examination.

In the foregoing description, the examination target presented by the target presentation apparatus 200 is confirmed for both the left and right eyes. Alternatively, the target presentation apparatus 200 may be configured to present the target only to the measured eye by utilizing polarization. For example, the target presentation apparatus 200 may be provided with a polarizing optical member to polarize the target light flux. Then, the target presentation apparatus 200 may be configured to present the examination target only to the measured eye by means of the polarizing optical element disposed in the optometry window. When an examination is performed in open-field state by thus utilizing polarization, the non-measured eye may also be fogged as described above. In this way, the influence of the non-measured eye on an examination of the measured eye can be suppressed.

<Display screen>

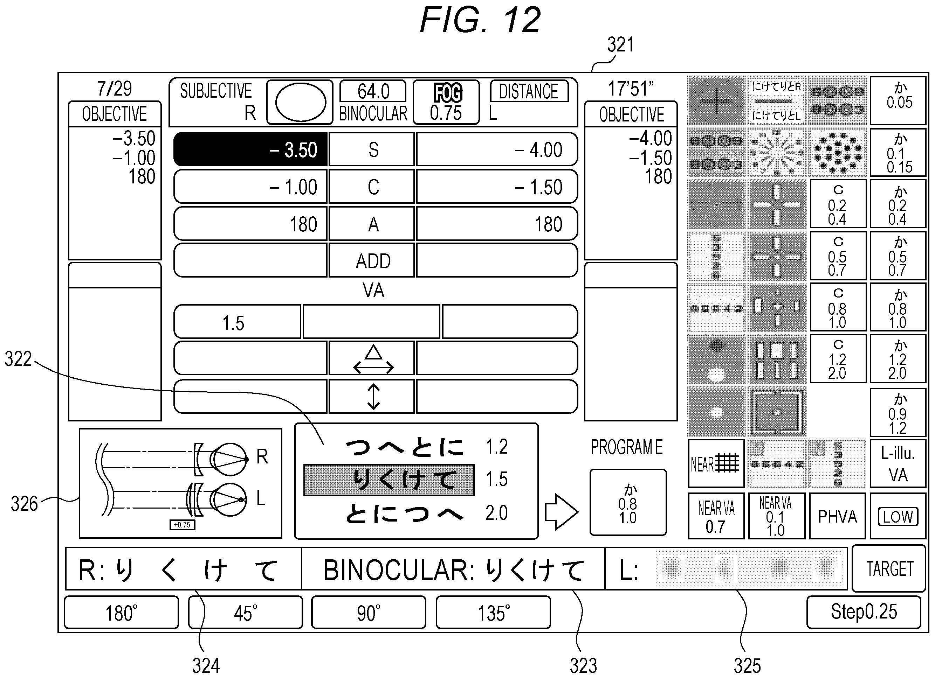

Display of the controller 300 according to the present example will be described. FIG. 12 is a diagram illustrating an example of a display screen 321 of the controller 300. In the display screen 321, a simulation image 323 indicating the view by both eyes of the examinee is displayed, for example.

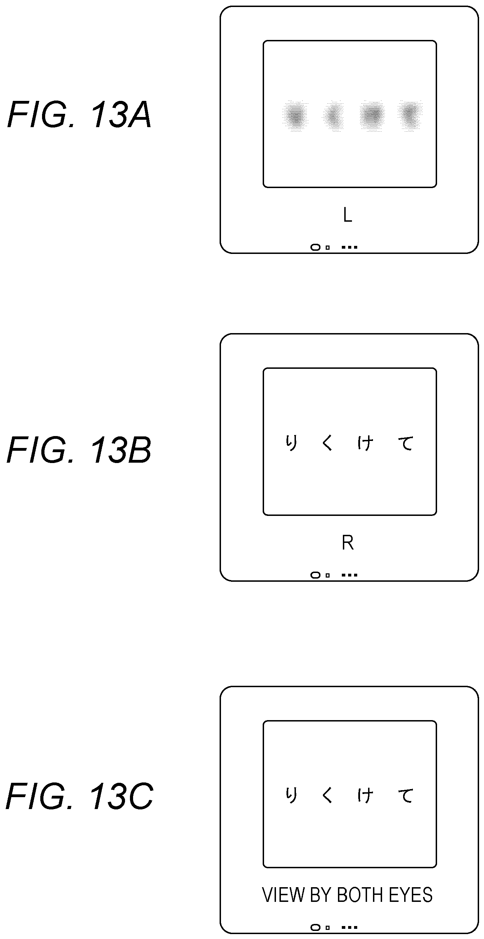

The simulation image 323 indicating the view by both eyes may be, for example, an image indicating the view by both eyes that is assumed when the examinee sees the examination target through the optometry window 160. For example, the simulation image 323 may be an image assuming that the retinal images obtained by the left and right eyes have been merged. When the retinal images are merged, often a sharper one of the retinal images obtained by the left and right eyes is preferentially visible. For example, FIG. 13A illustrates the view of the target by the left eye. FIG. 13B illustrates the view of the target by the right eye. FIG. 13C illustrates the view of the target by both eyes. As illustrated in FIGS. 13A to 13C, when the target is blurred to the left eye by fogging while the target is sharply visible to the right eye, the target is sharply visible to both eyes because the view by the right eye is given priority.

Accordingly, the control unit 370, on the basis of information about the visual acuity values of the examinee's left and right eyes, the corrective values of the left and right eyes, the amount of fog and the like, may display on the display unit 320 the view by one of the left and right eyes that has a sharper view as the binocular simulation image 323, for example. In the present example, when the non-measured eye is fogged, the view by the measured eye that is not fogged may be displayed as the view by both eyes on the display unit 320, for example. In this case, the control unit 370 may cause the display unit 320 to display a sharp examination target image visible to the measured eye as the simulation image 323.

The control unit 370 may display a simulation image indicating the view by each of the left and right eyes. For example, as illustrated in FIG. 12, the control unit 370 may cause the display unit 320 to display a simulation image 324 indicating the view by the right eye, a simulation image 325 indicating the view by the left eye, and the like. For example, when the non-measured eye is fogged in a monocular examination, the control unit 370 may cause the display unit 320 to display a blurred examination target image as the simulation image of the view on the non-measured eye side. For example, the control unit 370 may cause the display unit 320 to display an image that has been subjected to an obscuring process and the like in accordance with the amount of fog. The image displayed on the display unit 320 as the simulation image may be stored in the storage unit 340 and the like in advance, or generated by the control unit 370.

The control unit 370 may modify the degree of blurring of the simulation images 323, 324, and 325 in association with a modification of the corrective power and/or the amount of fog and the like. For example, the control unit 370 may modify the simulation image to a more blurred image when the amount of fog is increased. The control unit 370 may modify the simulation image to a sharper image when the amount of fog is reduced. In this way, the examiner can easily confirm a change in the view to the examinee by a modification of the corrective power and/or the amount of fog.

The control unit 370 may cause the display unit 320 to display an examination target (target image) 322 of the examination target being displayed on the target presentation unit 210. The examiner, by comparing the examination target (target image) 322 with other simulation images, can know whether the target is sharply visible to the examinee.

Thus, by causing the display unit 320 to display the simulation image 323 indicating the view by both eyes, it can be easily confirmed how the target is being visible to the examinee. Typically, when an examination is performed by fogging the examinee's eye, the examiner may confirm the view of the target by looking into the optometry apparatus 100. In this case, due to the influence of the examiner's binocular abnormality or the like, sometimes the view by the examiner may differ from the view by the examinee during measurement. As a result, it may become difficult for the examiner to know the view by the examinee (the examinee's eye). Accordingly, in the present example, instead of displaying a monocular simulation image to each eye, the control unit 370 causes the display unit 320 to display an image assuming that the retinal images respectively obtained by the left and right eyes have been merged. In this way, the examiner can easily know the view by the examinee (i.e., to share the view by the examinee with the examinee). In addition, by having the simulation image indicating the view by the examinee displayed, the examiner can perform an examination while knowing the view by the examinee.

The control unit 370 may perform, with respect to the image displayed as a simulation image in the case of fogging, a process for decreasing the brightness in accordance with the corrective power and/or an obscuring process of increasing an exposure value and the like. The obscuring process may be, for example, a process of changing or mixing colors, a color removal process, or brightness change. The control unit 370 may also acquire an image captured with optical obscuration, and cause the display unit 320 to display the image as a simulation image in the case of fogging.

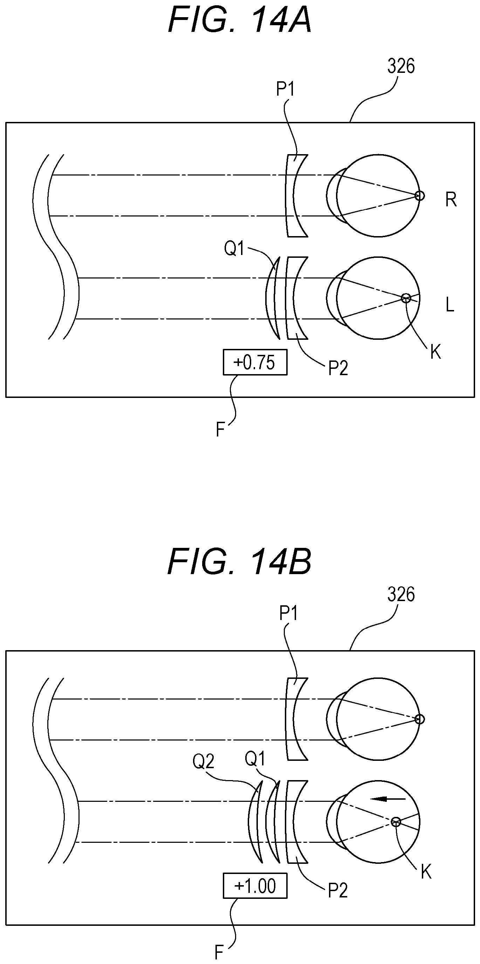

The control unit 370 may cause the display unit 320, for example, to display an eye chart 326, as illustrated in FIG. 12. The eye chart 326 is, for example, a light ray chart indicating how the target light flux enters the measured eye and the non-measured eye being fogged. FIGS. 14A and 14B illustrate the eye chart 326 as enlarged. As illustrated in these figures, the eye chart 326 includes an illustration of the examinee's eyes and lenses. As illustrated in FIG. 14A, for example, the eye chart has a corrective lens (for example, a concave lens) PI disposed for the measured eye, illustrating how the target light flux is focused on the retina. On the other hand, the eye chart also has a corrective lens P2 and a fogging lens (for example, a convex lens) Q1 disposed for the non-measured eye, illustrating how the target light flux is focused forwardly of the retina. In this way, the eye chart 326 is displayed on the display unit 320, whereby the examiner can easily assume the view by the examinee. The control unit 370 may display the amount of fog F near the fogging lens Q1.

Similarly to the above simulation image, the control unit 370 may modify the image forming position of the target light flux in the eye chart 326 in association with a modification of the lens power disposed in the optometry window 160. For example, the control unit 370, when the amount of fog applied to the examinee's eye is increased, may modify the eye chart 326 so as to dispose an additional fogging lens Q2 for the non-measured eye, and to move the image forming position K of the target light flux forward (see FIG. 14B). Conversely, the control unit 370, when the amount of fog is decreased, may modify the eye chart 326 so as to decrease the number of fogging lenses disposed for the non-measured eye, and to move the image forming position K of the target light flux toward the fundus. In this case, the control unit 370 may also modify the numerical value of the amount of fog F that is displayed.

In this way, the eye chart 326 is modified in accordance with the modification of the amount of fog, whereby a change in the view by the examinee in accordance with a power change can be easily confirmed. Accordingly, the examiner can easily confirm whether the degree of blurring in the view by the examinee has been increased or decreased by a power modification. The examiner can also describe the content of examination to the examinee in an easily understandable manner.

In the present example, the control unit 370 causes the display screen 321 to display the examination target 322; the binocular-view simulation image 323; the right-eye view simulation image 324; the left-eye view simulation image 325; and the eye chart 326. The examiner, by confirming the relationship of at least any of these displays, can more easily know the view by the examinee. However, the control unit 370 may cause the display screen 321 to display at least any of the examination target 322; the binocular-view simulation image 323; the right-eye view simulation image 324; the left-eye view simulation image 325; and the eye chart 326.

In the foregoing description, the control unit 370 causes the display unit 320 of the controller 300 to display the simulation image 323, the eye chart 326 and the like. However, this is not a limitation. For example, the control unit 370 may cause an optometry-information display apparatus (for example, a tablet terminal, a smartphone, a notebook PC, a desktop PC, and other display terminals) to display the simulation image, the eye chart and the like indicating the view. In this way, even when the optometry apparatus 100 is not actually used, such as when implementing an operation training for the optometry apparatus 100, for example, the instructor can easily describe the view at the time of fogging and the like to students.

The targeted visual acuity value may be a visual acuity value at the time of fogging the eye that is not measured (referred to as non-measured eye), so that, as a result, the visual acuity of the non-measured eye may be limited by fogging the non-measured eye.

The control unit 370 may determine the amount of fog on the basis of tables such as Table 1 and Table 2, for example. Table 1 may show the relationship between the targeted visual acuity value at the time of fogging and the spherical power A1 added to apply fogging with the targeted visual acuity value from a state of correction with an auto refractometer value (objective measurement value). Table 2 may show the correspondence relationship between the visual acuity value at the time of visual acuity measurement in a state of correction with an auto refractometer value, and the spherical power A2 corresponding to the degree of blurring at the time of seeing the target with the visual acuity value.

For example, the control unit 370 may determine the amount of fog F by substituting numerical values respectively corresponding to the visual acuity value of the examinee and a fog parameter into expression (1). Expression (1) may be an expression for determining the amount of fog F by subtracting, from the spherical power A1 corresponding to the targeted visual acuity value, the magnitude of the spherical power A2 obtained by converting the degree of blurring in a state of correction with an auto refractometer value.

For example, when the targeted visual acuity value of 0.7 is input by the examiner, the control unit 370 may apply a fog to such an extent that the target for visual acuity of 0.7 is visible with the non-measured eye. The control unit 370 may determine the spherical power A1 with respect to the visual acuity value of 0.7 from Table 1, and the spherical power A2 corresponding to the visual acuity value obtained by a visual acuity measurement from Table 2, and substitute the spherical powers into expression (1). In this case, if the result of visual acuity measurement is a visual acuity of 0.9, A1 = 0.75D and A2 = $\pm 0.25$. Accordingly, expression (1) yields F = 0.50.

In this way, the control unit 370, when the examinee's visual acuity is small (for example, less than 1.0), indicating an already blurred visible state, may add, to the non-measured eye, the amount of fog F obtained by subtracting the spherical power A2 from the spherical power A1 corresponding to the targeted visual acuity value at the time of fogging (see FIG. 8). That is, the control unit 370 may determine the final amount of fog F by correcting a reference power with a correction power. In the present example, the reference may be provided by the spherical power A1 required to lower the estimated visual acuity value (for example, 1.0 or more), estimated in a state of correction with an objective measurement value, to the targeted visual acuity value. Then, the spherical power A2 for correction may be determined on the basis of the difference between the examinee's actual visual acuity value and the estimated visual acuity value in the state of correction with the objective measurement value.

The present embodiment may be an optometry apparatus, an optometry program, an optometry-information display apparatus, or an optometry-information display program as set forth below.

A first optometry apparatus is an optometry apparatus for examining an examinee's eye, the optometry apparatus including a pair of right and left lens units which are disposed in an optical path of a target light flux projected onto the examinee and which change the optical characteristics of the target light flux; a drive means for driving the lens units; a control means for controlling driving of the drive means; and an acquisition means for acquiring a targeted visual acuity value at the time of fogging. The control means determines the amount of fog for fogging the examinee's eye, on the basis of the targeted visual acuity value acquired by the acquisition means.

A second optometry apparatus is the first optometry apparatus configured such that the acquisition means additionally acquires a visual acuity value of the examinee, and the control means determines the amount of fog, on the basis of the targeted visual acuity value and the visual acuity value of the examinee acquired by the acquisition means.

A third optometry apparatus is the first or the second optometry apparatus configured such that the acquisition means, on the basis of an operation signal output from an operation means in accordance with an operation by the examiner, acquires the targeted visual acuity value.

A fourth optometry apparatus is any of the first to the third optometry apparatus configured such that the pair of right and left lens units each includes at least an auto cross cylinder lens, the auto cross cylinder lens changes the optical characteristics of the target light flux traveling on a first optical path serving as the optical path of the target light flux projected onto the examinee's right eye, and a second optical path serving as the optical path of the target light flux projected onto the examinee's left eye, and the control means controls the driving of the drive means so that the separate directions of an examination target observed via the auto cross cylinder lens disposed in each of the first optical path and the second optical path become the same direction between the first optical path and the second optical path.

A fifth optometry apparatus is the fourth optometry apparatus configured such that the control means controls the driving of the drive means so that the auto cross cylinder lenses are disposed at the same axial angle in the first optical path and the second optical path.

A sixth optometry apparatus is the fourth or the fifth optometry apparatus configured such that the drive means further includes a rotation drive means for modifying the respective rotation angles of lenses disposed in the first optical path and the second optical path, and the control means, by controlling the driving of the rotation drive means, causes the auto cross cylinder lenses to rotate in conjunction with each other so that the axial angles of the auto cross cylinder lenses disposed in the first optical path and the second optical path are maintained to be the same between the first optical path and the second optical path.

A seventh optometry apparatus is the sixth optometry apparatus configured such that the control means, when rotating the auto cross cylinder lenses using the rotation drive means, align rotation directions and rotation amounts of the auto cross cylinder lenses respectively disposed in the first optical path and the second optical path.

An eighth optometry apparatus is the sixth or the seventh optometry apparatus configured such that the control means, when rotating the auto cross cylinder lenses using the rotation drive means, aligns rotational speeds of the auto cross cylinder lenses respectively disposed in in the first optical path and the second optical path.

A ninth optometry apparatus is any of the first to the eighth optometry apparatus further including a display means for displaying optometry information of the examinee's eye. The control means causes the display means to display a binocular simulation image indicating the view when the examination target is viewed by both eyes.

A tenth optometry apparatus is the ninth optometry apparatus configured such that the control means causes the display means to additionally display a monocular simulation image being at least one of a right-eye simulation image indicating the view of the examination target as viewed by the right eye and a left-eye simulation image indicating the view of the examination target as viewed by the left eye.

An eleventh optometry apparatus is the tenth optometry apparatus configured such that the control means sets the sharpness of at least one of the binocular simulation image and the monocular simulation image, on the basis of the visual acuity value of the examinee's eye.

A twelfth optometry apparatus is the eleventh optometry apparatus configured such that the visual acuity value of the examinee's eye is a visual acuity value estimated when the examinee's eye is fogged.

A thirteenth optometry apparatus is any one of the ninth to the twelfth optometry apparatus configured such that the control means sets the sharpness of the binocular simulation image on the basis of the higher of the right and left monocular visual acuity values.

A fourteenth optometry apparatus is any of the ninth to the thirteenth optometry apparatus configured such that the control means changes the sharpness of the simulation image in accordance with a power change in a corrective optical system for the examinee's eye corrected by a corrective means.

A first optometry program is an optometry program that is executed in an optometry apparatus for examining an examinee's eye, and that, by being executed by a processor of the optometry apparatus, causes the optometry apparatus to execute: an acquisition step of acquiring a targeted visual acuity value at the time of fogging and a visual acuity value of the examinee's eye; and, on the basis of the targeted visual acuity value and the measured visual acuity value, a determination step of determining the amount of fog to be added to fog the examinee's eye.

A fifteenth optometry apparatus is an optometry apparatus for examining an examinee's eye and provided with: a pair of right and left lens units which are disposed in an optical path of a target light flux projected onto the examinee, and which change the optical characteristics of the target light flux; a control means for controlling the driving of the lens units; and an acquisition means for acquiring a targeted visual acuity value at the time of fogging. The control means, on the basis of the targeted visual acuity value acquired by the acquisition means, determines the amount of fog for fogging the examinee's eye.

A sixteenth optometry apparatus is the fifteenth optometry apparatus configured such that the acquisition means additionally acquires a visual acuity value of the examinee, and the control means, on the basis of the targeted visual acuity value and the visual acuity value of the examinee acquired by the acquisition means, determines the amount of fog.

A seventeenth optometry apparatus is the fifteenth or the sixteenth optometry apparatus configured such that the acquisition means, on the basis of an operation signal output from an operation means in accordance with an operation by the examiner, acquires the targeted visual acuity value.

An eighteenth optometry apparatus is an optometry apparatus for examining an examinee's eye and provided with: a pair of right and left lens units which are disposed in an optical path of a target light flux projected onto the examinee, and which change the optical characteristics of the target light flux; a control means for controlling the driving of the lens units; and an acquisition means for acquiring a visual acuity value of the examinee. The control means, in accordance with the visual acuity value acquired by the acquisition means, corrects the amount of fog for fogging the examinee's eye.

A second optometry program is an optometry program which is executed in an optometry apparatus for examining an examinee's eye, and which, by being executed by a processor of the optometry apparatus, causes the optometry apparatus to execute: an acquisition step of acquiring a targeted visual acuity value at the time of fogging and a visual acuity value of the examinee's eye; and a determination step of determining, on the basis of the targeted visual acuity value and the measured visual acuity value, the amount of fog to be added to fog the examinee's eye.

Typically, there is an optometry apparatus in which an auto cross cylinder lens is disposed on the measured-eye side. By comparing the view of an examination target doubly separated by the auto cross cylinder lens, an examination of the astigmatism direction and power of the examinee's eye (for example, a cross cylinder test) can be easily performed.

However, in the optometry apparatus, when an open-field examination using the auto cross cylinder lens is performed, it has been sometimes impossible to perform an appropriate examination due to a difference in the view of the examination target between the left and right eyes.

Another technical problem of the present disclosure in view of the typical problem is to provide an optometry apparatus and an optometry program which enable an appropriate open-field examination.

With regard to the technical problem, a nineteenth optometry apparatus is an optometry apparatus for examining the examinee's eye and provided with: a pair of right and left lens units which are respectively disposed in a first optical path serving as an optical path of a target light flux projected onto the examinee's right eye, and a second optical path serving as an optical path of the target light flux projected onto the examinee's left eye, and which change, using at least an auto cross cylinder lens, the optical characteristics of the target light flux in each of the first optical path and the second optical path; a drive means for driving the lens units; and a control means for controlling driving of the drive means. The control means controls the driving of the drive means so that the separate directions of an examination target observed via the auto cross cylinder lens disposed in each of the first optical path and the second optical path have the same direction between the first optical path and the second optical path.

A twentieth optometry apparatus is the nineteenth optometry apparatus configured such that the control means controls the driving of the drive means so that the auto cross cylinder lenses are disposed at the same axial angle in the first optical path and the second optical path.

A twenty-first optometry apparatus is the nineteenth or the twentieth optometry apparatus configured such that the drive means is provided with a rotation drive means for modifying the respective rotation angles of lenses disposed in the first optical path and the second optical path. The control means, by controlling the driving of the rotation drive means, causes the lenses to rotate in conjunction with each other so that the axial angles of the auto cross cylinder lenses disposed in the first optical path and the second optical path are maintained to be the same between the first optical path and the second optical path.

A twenty-second optometry apparatus is the twenty-first optometry apparatus configured such that the control means, when rotating the auto cross cylinder lenses using the rotation drive means, aligns the rotation directions and rotation amounts of the auto cross cylinder lenses respectively disposed in the first optical path and the second optical path.

A twenty-third optometry apparatus is the twenty-first or the twenty-second optometry apparatus configured such that the control means, when rotating the auto cross cylinder lenses using the rotation drive means, aligns the rotational speeds of the auto cross cylinder lenses respectively disposed in the first optical path and the second optical path.

A third optometry program is an optometry program which is executed in an optometry apparatus for examining an examinee's eye, and which, by being executed by a processor of the optometry apparatus, causes the optometry apparatus to execute a control step of controlling the driving of lens units so that, by the lens units, separate directions of an examination target, which is observed via an auto cross cylinder lens disposed in each of a first optical path serving as an optical path of a target light flux projected onto the examinee's right eye and a second optical path serving as an optical path of the target light flux projected onto the examinee's left eye, have the same direction between the first optical path and the second optical path.

When optometry is performed with the left and right eyes being differently corrected, as when the non-measured eye is fogged, it has been difficult for the examiner to visualize how the examination target is being viewed by the examinee.

In view of the typical problem, an additional technical problem of the present disclosure is to provide an optometry-information display apparatus and an optometry-information display program with which the view of the examination target by the examinee can be easily visualized.

With regard to this technical problem, a first optometry-information display apparatus is an optometry-information display apparatus for displaying optometry information of an examinee's eye, and is provided with a control means for causing a display means to display a binocular simulation image indicating the view when the examination target is viewed by both eyes.

A second optometry-information display apparatus is the first optometry-information display apparatus configured such that the control means causes the display means to additionally display a monocular simulation image being at least any of a right-eye simulation image indicating the view of the examination target as viewed by the right eye and a left-eye simulation image indicating the view of the examination target as viewed by the left eye.

A third optometry-information display apparatus is the second optometry-information display apparatus configured such that the control means sets a sharpness of at least one of the binocular simulation image and the monocular simulation image, on the basis of a visual acuity value of the examinee's eye.

A fourth optometry-information display apparatus is the third optometry-information display apparatus configured such that the visual acuity value of the examinee's eye is a visual acuity value estimated when the examinee's eye is fogged.

A fifth optometry-information display apparatus is any of the first to the fourth optometry-information display apparatus configured such that the control means sets a sharpness of the binocular simulation image on the basis of the higher of right and left monocular visual acuity values.

A sixth optometry-information display apparatus is any of the first to the fifth optometry-information display apparatus configured such that the control means changes a sharpness of the simulation image in accordance with a power change in a corrective optical system for the examinee's eye corrected by a corrective means.

A seventh optometry-information display apparatus is any of the first to the sixth optometry-information display apparatus configured such that the control means causes the display means to additionally display an eye chart having a drawing of at least the examinee's eye and an image forming position of a target light flux entering the examinee's eye.

An eighth optometry-information display apparatus is the seventh optometry-information display apparatus configured such that the eye chart includes a drawing of a corrective optical system disposed in front of the examinee's eye.

A ninth optometry-information display apparatus is the seventh or the eighth optometry-information display apparatus configured such that the control means changes the drawing of the eye chart in accordance with a change in the corrected state of the examinee's eye corrected by a corrective means.

A first optometry-information display program is an optometry-information display program which is executed in an optometry-information display apparatus for displaying optometry information of an examinee's eye, and which, by being executed by a processor of the optometry-information display apparatus, causes the optometry-information display apparatus to execute a display step of causing a display means to display a binocular simulation image indicating the view when the examination target is viewed by both eyes.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

**Claims**

1. An optometry apparatus (100) comprising:

   a pair of right and left lens units (110) disposed in an optical path of a target light flux projected onto an examinee's eye, configured for changing an optical characteristic of the target lifght flux;
   a drive means (130R, 130L, 150R, 150L) configured for driving the lens units (110);
   a control means (370) configured for controlling driving of the drive means (130R, 130L, 150R, 150L);
   an acquisition means (370) configured for acquiring a targeted visual acuity value at the time of fogging and to additionally aquire a measured visual acuity value of the examinee's eye; and
   an operation means (300), wherein the acquisition means is configured to acquire the targeted visual acuity value on the basis of an operation signal output from the operation means (300) in accordance with an operation by the examiner, wherein
   the control means (370) is configured to determine an amount of fog for fogging the examinee's eye on the basis of the targeted visual acuity value acquired by the acquisition means (370) and the measured visual acuity value of the examinee's eye acquired by the acquisition means (370), wherein

the control means (370) is configured to determine the amount of fog on the basis of a power corresponding to the targeted visual acuity value and a correction value corresponding to the visual acuity value of the examinee, wherein

the correction value is a power obtained by conversion of the degree of blurring corresponding to the visual acuity value of the examinee, wherein

the correction value is obtained by converting a displacement between the image forming position of the examination target that enters the examinee's eye that has been corrected and the retinal position of the examinee's eye into a lens power.

2. The optometry apparatus (100) according to claim 1, wherein
the pair of right and left lens units (110) each includes at least an auto cross cylinder lens (Ac),
the pair of right and left lens units (110) is configured to change the optical characteristic of the target light flux travelling on a first optical path serving as an optical path of the target light flux projected onto the examinee's right eye, and a second optical path serving as an optical path of the target light flux projected onto the examinee's left eye with the auto cross cylinder lens (Ac), and
the control means (370) are configured to control the driving of the drive means (130R, 130L, 150R, 150L) so that separate directions of an examination target observed via the auto cross cylinder lens (Ac) disposed in each of the first optical path and the second optical path become the same direction between the first optical path and the second optical path.

3. The optometry apparatus (100) according to claim 2, wherein
the control means (370) is configured to control the driving of the drive means (130R, 130L, 150R, 150L) so that the axes of the auto cross cylinder lenses (Ac) have the same axial angle between the first optical path and the second optical path.

4. The optometry apparatus (100) according to claim 2 or 3, wherein
the drive means (130R, 130L, 150R, 150L) further includes a rotation drive means (150R, 150L) configured for modifying the respective rotation angles of the auto cross cylinder lenses (Ac) respectively disposed in the first optical path and the second optical path, and
the control means (370) is configured to, by controlling the driving of the rotation drive means (150R, 150L), rotate the auto cross cylinder lenses (Ac) in conjunction with each other so that the axial angles of the auto cross cylinder lenses (Ac) respectively disposed in the first optical path and the second optical path are maintained to be the same between the first optical path and the second optical path.

5. The optometry apparatus (100) according to claim 4, wherein
the control means (370) is configured to, when rotating the auto cross cylinder lenses (Ac) using the rotation drive means (150R, 150L), align the rotation directions and rotation amounts of the auto cross cylinder lenses (Ac) respectively disposed in the first optical path and the second optical path.

6. The optometry apparatus (100) according to claim 4 or 5, wherein
the control means (370) is configured to, when rotating the auto cross cylinder lenses (Ac) using the rotation drive means (150R, 150L), align the rotational speeds of the auto cross cylinder lenses (Ac) respectively disposed in the first optical path and the second optical path.

7. The optometry apparatus (100) according to any one of the preceding claims, further comprising
a display means (320) configured for displaying optometry information of the examinee's eye, wherein
the control means (370) is configured to cause the display means (320) to display a binocular simulation image (323) indicating a view of the examination target as viewed by both eyes.

8. The optometry apparatus (100) according to claim 7, wherein
the control means (370) is configured to cause the display means (320) to additionally display a monocular simulation image (324, 325) being at least one of a right-eye simulation image (324) indicating a view of the examination target as viewed by the right eye, and a left-eye simulation image (325) indicating a view of the examination target as viewed by the left eye.

9. The optometry apparatus (100) according to claim 8, wherein
the control means (370) is configured to set sharpness of at least one of the binocular simulation image (323) and the monocular simulation image (324, 325) on the basis of the visual acuity value of the examinee's eye.

10. The optometry apparatus (100) according to claim 9, wherein
the visual acuity value of the examinee's eye is an estimated visual acuity value of the examinee's eye being fogged.

11. The optometry apparatus (100) according to any one of claims 7 to 10, wherein
the control means (370) is configured to set sharpness of the binocular simulation image (323) based on the higher of the right and left monocular visual acuity values.

12. The optometry apparatus (100) according to any one of claims 7 to 11, further comprising
a corrective means (110) configured for correcting the examinee's eye, wherein
the control means (370) is configured to change the sharpness of the simulation image in accordance with a power change in a corrective optical system of the corrective means (110).

13. An optometry program which, when being executed by the optometry apparatus (100) as defined in claim 1, is adapted to cause the optometry apparatus (100) to implement:

an acquisition step of acquiring a targeted visual acuity value at the time of fogging and a measured visual acuity value of the examinee's eye; and
a determination step of determining, on the basis of the targeted visual acuity value and the measured visual acuity value, an amount of fog to be added when fogging the examinee's eye,
and determining the amount of fog on the basis of a power corresponding to the targeted visual acuity value and a correction value corresponding to the visual acuity value of the examinee, wherein
the correction value is a power obtained by conversion of the degree of blurring corresponding to the visual acuity value of the examinee, werhein
the correction value is obtained by converting a displacement between the image forming position of the examination target that enters the examinee's eye that has been corrected and the retinal position of the examinee's eye into a lens power.

## Patentansprüche

1. Eine Optometrievorrichtung (100), welche die folgenden Merkmale aufweist:

ein Paar rechter und linker Linseneinheiten (110), die in einem Strahlengang eines auf ein Auge eines Probanden projizierten Ziellichtstromes angeordnet sind, dazu ausgebildet, eine optische Eigenschaft des Ziellichtstromes zu ändern;
eine Antriebseinrichtung (130R, 130L, 150R, 150L), die dazu ausgebildet ist, die Linseneinheiten (110) anzutreiben;
eine Steuereinrichtung (370), die dazu ausgebildet ist, ein Antreiben der Antriebseinrichtung (130R, 130L, 150R, 150L) zu steuern;
eine Erfassungseinrichtung (370), die dazu ausgebildet ist, einen anvisierten Sehschärfewert zum Zeitpunkt einer Verschleierung zu erfassen und zusätzlich einen gemessenen Sehschärfewert des Auges des Probanden zu erfassen; und
eine Betätigungseinrichtung (300), wobei die Erfassungseinrichtung dazu ausgebildet ist, den anvisierten Sehschärfewert auf der Basis eines gemäß einer Betätigung durch den Prüfer von der Betätigungseinrichtung (300) ausgegebenen Betätigungssignals zu erfassen, wobei
die Steuereinrichtung (370) dazu ausgebildet ist, eines Schleierbetrages zur Verschleierung des Auges des Probanden auf der Basis des durch die Erfassungseinrichtung (370) erfassten anvisierten Sehschärfewertes und des durch die Erfassungseinrichtung (370) erfassten gemessenen Sehschärfewertes des Auges des Probanden zu bestimmen, wobei
die Steuereinrichtung (370) dazu ausgebildet ist, den Schleierbetrag auf der Basis einer dem anvisierten Sehschärfewert entsprechenden Brechkraft und eines dem Sehschärfewert des Probanden entsprechenden Korrekturwertes zu bestimmen, wobei
der Korrekturwert eine Brechkraft ist, die durch Umwandlung des dem Sehschärfewert des Probanden entsprechenden Unschärfegrades erhalten wird, wobei
der Korrekturwert durch Umwandeln einer Verschiebung zwischen der Bilderstellungsposition des Untersuchungsgegenstandes, der in das korrigierte Auge des Probanden eintritt, und der Netzhautposition des Auges des Probanden in eine Linsenbrechkraft erhalten wird.

2. Die Optometrievorrichtung (100) gemäß Anspruch 1, wobei
das Paar rechter und linker Linseneinheiten (110) jeweils zumindest eine Autokreuzzylinderlinse (Ac) umfasst,
wobei das Paar rechter und linker Linseneinheiten (110) dazu ausgebildet ist, die optische Eigenschaft des Ziellichtstromes, der sich auf einem ersten Strahlengang, welcher als ein Strahlengang des auf das rechte Auge des Probanden projizierten Ziellichtstromes dient, und sich auf einem zweiten Strahlengang bewegt, der als Strahlengang des auf das linke Auge des Probanden projizierten Ziellichtflusses dient, mit der Autokreuzzylinderlinse (Ac) zu ändern, und
wobei die Steuereinrichtung (370) dazu ausgebildet ist, das Antreiben der Antriebseinrichtung (130R, 130L, 150R, 150L) zu steuern, so dass getrennte Richtungen eines Untersuchungsgegenstandes, der über die Autokreuzzylinderlinse (Ac) beobachtet wird, welche in dem ersten Strahlengang und dem zweiten Strahlengang angeordnet ist, dieselbe Richtung zwischen dem ersten Strahlengang und dem zweiten Strahlengang werden.

3. Die Optometrievorrichtung (100) gemäß Anspruch 2, wobei
die Steuereinrichtung (370) dazu ausgebildet ist, das Antreiben der Antriebseinrichtung (130R, 130L, 150R, 150L) zu steuern, so dass die Achsen der Autokreuzzylinderlinsen (Ac) denselben Axialwinkel zwischen dem ersten Strahlengang und dem zweiten Strahlengang aufweisen.

4. Die Optometrievorrichtung (100) gemäß Anspruch 2 oder 3, wobei
die Antriebseinrichtung (130R, 130L, 150R, 150L) ferner eine Rotationsantriebseinrichtung (150R, 150L) umfasst, die dazu ausgebildet ist, die jeweiligen Rotationswinkel der Autokreuzzylinderlinsen (Ac), die jeweils in dem ersten Strahlengang und dem zweiten Strahlengang angeordnet sind, zu modifizieren, und
wobei die Steuereinrichtung (370) dazu ausgebildet ist, durch das Steuern des Antriebes der Rotationsantriebseinrichtung (150R, 150L) die Autokreuzzylinderlinsen (Ac) in Verbindung miteinander zu drehen, so dass die Axialwinkel der Autokreuzzylinderlinsen (Ac), die jeweils in dem ersten Strahlengang und dem zweiten Strahlengang angeordnet sind, gleich gehalten werden, so dass dieselben zwischen dem ersten Strahlengang und dem zweiten Strahlengang gleich sind.

5. Die Optometrievorrichtung (100) gemäß Anspruch 4, wobei
die Steuereinrichtung (370) dazu ausgebildet ist, beim Rotieren der Autokreuzzylinderlinsen (Ac) unter Verwendung der Rotationsantriebseinrichtung (150R, 150L) die Rotationsrichtungen und Rotationsgrößen der jeweils in dem ersten Strahlengang und dem zweiten Strahlengang angeordneten sind Autokreuzzylinderlinsen (Ac) auszurichten.

6. Die Optometrievorrichtung (100) gemäß Anspruch 4 oder 5, wobei die Steuereinrichtung (370) dazu ausgebildet ist, beim Rotieren der Autokreuzzylinderlinsen (Ac) unter Verwendung der Rotationsantriebseinrichtung (150R, 150L) die Rotationsgeschwindigkeiten der jeweils in dem ersten Strahlengang und dem zweiten Strahlengang angeordneten Autokreuzzylinderlinsen (Ac) auszurichten.

7. Die Optometrievorrichtung (100) gemäß einem der vorhergehenden Ansprüche, die ferner folgende Merkmale aufweist:

   eine Anzeigeeinrichtung (320), die dazu ausgebildet ist, Optometrieinformationen des Auges des Probanden anzuzeigen, wobei
   die Steuereinrichtung (370) dazu ausgebildet ist, zu bewirken, dass die Anzeigeeinrichtung (320) ein binokulares Simulationsbild (323) anzeigt, welches eine Ansicht des Untersuchungsgegenstandes wie durch beide Augen gesehen angibt.

8. Die Optometrievorrichtung (100) gemäß Anspruch 7, wobei
die Steuereinrichtung (370) dazu ausgebildet ist, zu bewirken, dass die Anzeigeeinrichtung (320) zusätzlich ein monookulares Simulationsbild (324, 325) anzeigt, welches ein Rechtes-Auge-Simulationsbild (324), das eine Ansicht des Untersuchungsgegenstandes wie durch das rechte Auge gesehen angibt, und/oder ein Linkes-Auge-Simulationsbild (325) ist, das eine Ansicht des Untersuchungsgegenstandes wie durch das linke Auge gesehen angibt.

9. Die Optometrievorrichtung (100) gemäß Anspruch 8, wobei
die Steuereinrichtung (370) dazu ausgebildet ist, eine Schärfe des binokularen Simulationsbildes (323) und/oder des monookularen Simulationsbildes (324, 325) auf der Basis des Sehschärfewertes des Auges des Probanden einzustellen.

10. Die Optometrievorrichtung (100) gemäß Anspruch 9, wobei

der Sehschärfewert des Auges des Probanden ein geschätzter Sehschärfewert des Auges des Probanden ist, wenn dieses verschleiert ist.

**11.** Die Optometrievorrichtung (100) gemäß einem der Ansprüche 7 bis 10, wobei die Steuereinrichtung (370) dazu ausgebildet ist, eine Schärfe des binokularen Simulationsbildes (323) auf der Basis des höheren des rechten und linken monookularen Sehschärfewertes einzustellen.

**12.** Die Optometrievorrichtung (100) gemäß einem der Ansprüche 7 bis 11, die ferner Folgendes aufweist:

eine Korrektureinrichtung (110), die dazu ausgebildet ist, das Auge des Probanden zu korrigieren, wobei die Steuereinrichtung (370) dazu ausgebildet ist, die Schärfe des Simulationsbildes gemäß einer Brechkraftänderung eines optischen Korrektursystems der Korrektureinrichtung (110) zu ändern.

**13.** Ein Optometrieprogramm, das dann, wenn dasselbe durch die Optometrievorrichtung (100) gemäß Anspruch 1 ausgeführt wird, dazu angepasst ist, zu bewirken, dass die Optometrievorrichtung (100) Folgendes implementiert:

einen Erfassungsschritt zum Erfassen eines anvisierten Sehschärfewertes zum Zeitpunkt einer Verschleierung und eines gemessenen Sehschärfewertes des Auges des Probanden; und
einen Bestimmungsschritt zum Bestimmen eines Schleierbetrages, der beim Verschleiern des Auges des Probanden hinzuzufügen ist, auf der Basis des anvisierten Sehschärfewertes und des gemessenen Sehschärfewertes, und
Bestimmen des Schleierbetrages auf der Basis einer dem anvisierten Sehschärfewert entsprechenden Brechkraft und eines dem Sehschärfewert des Probanden entsprechenden Korrekturwertes, wobei
der Korrekturwert eine Brechkraft ist, die durch Umwandlung des dem Sehschärfewert des Probanden entsprechenden Unschärfegrades erhalten wird, wobei
der Korrekturwert durch Umwandeln einer Verschiebung zwischen der Bilderstellungsposition des Untersuchungsgegenstandes, der in das korrigierte Auge des Probanden eintritt, und der Netzhautposition des Auges des Probanden in eine Linsenbrechkraft erhalten wird.

## Revendications

**1.** Appareil d'optométrie (100), comprenant:

une paire d'unités de lentilles droite et gauche (110) disposées sur un trajet optique d'un flux de lumière cible projeté sur l'oeil d'une personne examinée, configurées pour modifier une caractéristique optique du flux de lumière cible;
un moyen d'entraînement (130R, 130L, 150R, 150L) configuré pour entraîner les unités de lentille (110);
un moyen de commande (370) configuré pour commander l'entraînement du moyen d'entraînement (130R, 130L, 150R, 150L);
un moyen d'acquisition (370) configuré pour acquérir une valeur d'acuité visuelle ciblée au moment de la formation de brume et pour acquérir en outre une valeur d'acuité visuelle mesurée de l'œil de la personne examinée; et
un moyen d'actionnement (300), où le moyen d'acquisition est configuré pour acquérir la valeur d'acuité visuelle ciblée sur base d'un signal d'actionnement émis par le moyen d'actionnement (300) selon un actionnement effectué par l'examinateur, dans lequel
le moyen de commande (370) est configuré pour déterminer une quantité de brume pour voiler l'œil de la personne examinée sur base de la valeur d'acuité visuelle ciblée acquise par le moyen d'acquisition (370) et de la valeur d'acuité visuelle mesurée de l'œil de la personne examinée acquise par le moyen d'acquisition (370), dans lequel
le moyen de commande (370) est configuré pour déterminer la quantité de brume sur base d'une puissance correspondant à la valeur d'acuité visuelle ciblée et d'une valeur de correction correspondant à la valeur d'acuité visuelle de la personne examinée, dans lequel
la valeur de correction est une puissance obtenue par conversion du degré de flou correspondant à la valeur d'acuité visuelle de la personne examinée, dans lequel
la valeur de correction est obtenue en convertissant un déplacement entre la position de formation d'image de la cible d'examen qui pénètre dans l'œil de la personne examinée qui a été corrigée et la position de la rétine de l'œil de la personne examinée en une puissance de lentille.

**2.** Appareil d'optométrie (100) selon la revendication 1, dans lequel
la paire d'unités de lentille droite et gauche (110) comportent, chacune, au moins une lentille cylindrique auto-croisée (Ac),
la paire d'unités de lentilles droite et gauche (110) est configurée pour modifier la caractéristique optique du flux de lumière cible se déplaçant sur un premier trajet optique servant de trajet optique du flux de lumière cible projeté sur l'œil droit de la personne examinée, et un deuxième trajet optique servant de trajet optique du flux de lumière cible projeté sur l'œil gauche du personne examinée avec la lentille auto-cylindrique (Ac), et
le moyen de commande (370) est configuré pour commander l'entraînement du moyen d'entraînement (130R, 130L, 150R, 150L) de sorte que différentes directions d'une cible d'examen observée par l'intermédiaire de la lentille cylindrique auto-croisée (Ac) disposée sur chacun du premier trajet optique et du deuxième trajet optique deviennent la même direction entre le premier trajet optique et le deuxième trajet optique.

**3.** Appareil d'optométrie (100) selon la revendication 2, dans lequel
le moyen de commande (370) est configuré pour commander l'entraînement du moyen d'entraînement (130R, 130L, 150R, 150L) de sorte que les axes des lentilles cylindriques auto-croisées (Ac) présentent le même angle axial entre le premier trajet optique et le deuxième trajet optique.

**4.** Appareil d'optométrie (100) selon la revendication 2 ou 3, dans lequel
le moyen d'entraînement (130R, 130L, 150R, 150L) comporte par ailleurs un moyen d'entraînement en rotation (150R, 150L) configuré pour modifier les angles de rotation respectifs des lentilles cylindriques auto-croisées (Ac) disposées respectivement sur le premier trajet optique et le deuxième optique chemin, et
le moyen de commande (370) est configuré pour faire tourner, lors de la commande de l'entraînement du moyen d'entraînement en rotation (150R, 150L), les lentilles cylindriques auto-croisées (Ac) ensemble l'une avec l'autre de sorte que les angles axiaux des lentilles cylindriques auto-croisées (Ac) disposées respectivement sur le premier trajet optique et le deuxième trajet optique soient maintenus identiques entre le premier trajet optique et le deuxième trajet optique.

**5.** Appareil d'optométrie (100) selon la revendication 4, dans lequel
le moyen de commande (370) est configuré pour aligner, lors de la rotation des lentilles cylindriques auto-croisées (Ac) à l'aide du moyen d'entraînement en rotation (150R, 150L), les directions de rotation et les quantités de rotation des lentilles cylindriques auto-croisées (Ac) disposées respectivement sur le premier trajet optique et le deuxième trajet optique.

**6.** Appareil d'optométrie (100) selon la revendication 4 ou 5, dans lequel
le moyen de commande (370) est configuré pour aligner, lors de la rotation des lentilles cylindriques auto-croisées (Ac) à l'aide du moyen d'entraînement en rotation (150R, 150L), les vitesses de rotation des lentilles cylindriques auto-croisées (Ac) disposées respectivement sur le premier trajet optique et le deuxième trajet optique.

**7.** Appareil d'optométrie (100) selon l'une quelconque des revendications précédentes, comprenant par ailleurs un moyen d'affichage (320) configuré pour afficher les informations d'optométrie de l'œil de la personne examinée, dans lequel
le moyen de commande (370) est configuré pour amener le moyen d'affichage (320) à afficher une image de simulation binoculaire (323) indiquant une vue de la cible d'examen vue par les deux yeux.

**8.** Appareil d'optométrie (100) selon la revendication 7, dans lequel
le moyen de commande (370) est configuré pour amener le moyen d'affichage (320) à afficher en outre une image de simulation monoculaire (324, 325) qui est au moins l'une parmi une image de simulation de l'œil droit (324) indiquant une vue de la cible d'examen vue par l'œil droit, et une image de simulation de l'œil gauche (325) indiquant une vue de la cible d'examen vue par l'œil gauche.

**9.** Appareil d'optométrie (100) selon la revendication 8, dans lequel
le moyen de commande (370) est configuré pour régler la netteté d'au moins l'une parmi l'image de simulation binoculaire (323) et l'image de simulation monoculaire (324, 325) sur base de la valeur d'acuité visuelle de l'œil de la personne examinée.

**10.** Appareil d'optométrie (100) selon la revendication 9, dans lequel
la valeur d'acuité visuelle de l'œil de la personne examinée est une valeur estimée d'acuité visuelle de l'œil de la personne examinée qui est voilé.

**11.** Appareil d'optométrie (100) selon l'une quelconque des revendications 7 à 10, dans lequel
le moyen de commande (370) est configuré pour régler la netteté de l'image de simulation binoculaire (323) sur base de la plus élevée des valeurs d'acuité visuelle monoculaire droite et gauche.

**12.** Appareil d'optométrie (100) selon l'une quelconque des revendications 7 à 11, comprenant par ailleurs
un moyen de correction (110) configuré pour corriger l'œil de la personne examinée, dans lequel
le moyen de commande (370) est configuré pour modifier la netteté de l'image de simulation selon une modification de puissance dans un système optique de correction du moyen de correction (110).

**13.** Programme d'optométrie qui, lorsqu'il est exécuté par l'appareil d'optométrie (100) tel que défini dans la revendication 1, est adapté pour amener l'appareil d'optométrie (100) à mettre en œuvre:

une étape d'acquisition consistant à acquérir une valeur d'acuité visuelle ciblée au moment de la formation de brume et une valeur d'acuité visuelle mesurée de l'œil de la personne examinée; et
une étape de détermination consistant à déterminer, sur base de la valeur d'acuité visuelle ciblée et de la valeur d'acuité visuelle mesurée, une quantité de brume à ajouter lors de la brumisation de l'œil de la personne examinée,
et déterminer la quantité de brume sur base d'une puissance correspondant à la valeur d'acuité visuelle ciblée et d'une valeur de correction correspondant à la valeur d'acuité visuelle de la personne examinée, dans lequel
la valeur de correction est une puissance obtenue par conversion du degré de flou correspondant à la valeur d'acuité visuelle de la personne examinée, dans lequel
la valeur de correction est obtenue en convertissant un déplacement entre la position de formation d'image de la cible d'examen qui pénètre dans l'œil de la personne examinée qui a été corrigée et la position de la rétine de l'œil de la personne examinée en une puissance de lentille.

# FIG. 1

EP 3 175 776 B1

## FIG. 2

## FIG. 3

## FIG. 4

## FIG. 5

# FIG. 6

```
        START

S1 —— ACQUIRE FOG PARAMETER

S2 —— PRELIMINARY MEASUREMENT

S3 —— CALCULATE FOG AMOUNT

S4 —— ADD POWER OF FOG

S5 —— MONOCULAR EXAMINATION

S6 —— BINOCULAR BALANCE TEST

S7 —— MEASURE BINOCULAR
        COMPLETE CORRECTIVE VALUE

         END
```

## FIG. 7

360

TARGETED VISUAL ACUITY
DURING FOGGING

361

△

0.6

0.7

0.8

▽

362

363

SET

## FIG. 8

AMOUNT OF FOG FOR (A1)

STATE OF (A2)

(A1)−|(A2)|

FIG. 9A

FIG. 9B

FIG. 9C

FIG. 10A

FIG. 10B

FIG. 10C

# FIG. 11

# FIG. 12

EP 3 175 776 B1

FIG. 13A

L

FIG. 13B

R

FIG. 13C

VIEW BY BOTH EYES

# FIG. 14A

# FIG. 14B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11285470 A
- JP H11285470 A
- JP H11285486 A
- JP H08238220 A